(19) Europäisches Patentamt European Patent Office Office européen des brevets

(11) EP 3 370 750 B1

## (12) EUROPEAN PATENT SPECIFICATION

(45) Date of publication and mention
of the grant of the patent:
**20.08.2025 Bulletin 2025/34**

(21) Application number: **16791604.8**

(22) Date of filing: **08.11.2016**

(51) International Patent Classification (IPC):
*A61K 36/31* (2006.01)    *A61K 35/20* (2006.01)
*A61K 35/744* (2015.01)    *A23L 33/135* (2016.01)
*A61P 1/04* (2006.01)    *A61P 1/10* (2006.01)
*A61P 1/12* (2006.01)    *A61P 1/14* (2006.01)
*A61P 1/16* (2006.01)    *A61P 3/06* (2006.01)
*A61P 9/12* (2006.01)    *A61P 17/00* (2006.01)
*A61P 17/10* (2006.01)    *A61P 19/02* (2006.01)
*A61K 35/747* (2015.01)    *A61P 25/00* (2006.01)
*A61P 29/00* (2006.01)    *A61P 37/02* (2006.01)
*A61P 37/08* (2006.01)    *A61P 31/04* (2006.01)

(52) Cooperative Patent Classification (CPC):
(C-Sets available)
**A23L 33/135; A61K 35/20; A61K 35/744;
A61K 35/747; A61K 36/31; A61P 1/04; A61P 1/10;
A61P 1/12; A61P 1/14; A61P 1/16; A61P 3/06;
A61P 9/12; A61P 17/00; A61P 17/10; A61P 19/02;**
(Cont.)

(86) International application number:
**PCT/EP2016/076952**

(87) International publication number:
**WO 2017/077139 (11.05.2017 Gazette 2017/19)**

(54) **COMBINED PRE- AND PRO-BIOTIC COMPOSITION**

KOMBINIERTE PRÄ- UND PROBIOTISCHE ZUSAMMENSETZUNG

COMPOSITION PRÉ- ET PRO-BIOTIQUE COMBINÉE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.11.2015 DK 201500700
29.02.2016 DK 201600128
20.07.2016 DK 201600431
14.09.2016 DK 201600537**

(43) Date of publication of application:
**12.09.2018 Bulletin 2018/37**

(73) Proprietor: **Fermbiotics Holding ApS
6622 Bække (DK)**

(72) Inventor: **LEGARTH, Jens, Høffner
6622 Bække (DK)**

(74) Representative: **Larsen & Birkeholm A/S
Banegårdspladsen 1
1570 Copenhagen V (DK)**

(56) References cited:
**EP-A1- 2 514 318        WO-A1-2004/073418
WO-A2-2014/131422        WO-A2-2014/206419**

- **Y. J. CHOI ET AL: "Effects of Dietary Fermented
Seaweed and Seaweed Fusiforme on Growth
Performance, Carcass Parameters and
Immunoglobulin Concentration in Broiler
Chicks", ASIAN-AUSTRALASIAN JOURNAL OF
ANIMAL SCIENCES., vol. 27, no. 6, 24 April 2014
(2014-04-24), KR, pages 862 - 870, XP055633537,
ISSN: 1011-2367, DOI: 10.5713/ajas.2014.14015**

**(Cont. next page)**

- MICHELA MAIFRENI ET AL: "Lactic acid fermentation of Brassica rapa : chemical and microbial evaluation of a typical Italian product ( brovada )", EUROPEAN FOOD RESEARCH AND TECHNOLOGY, vol. 218, no. 5, 1 April 2004 (2004-04-01), pages 469 - 473, XP055328504, ISSN: 1438-2377, DOI: 10.1007/ s00217-004-0877-6
- MANAS RANJAN SWAIN ET AL: "Fermented Fruits and Vegetables of Asia: A Potential Source of Probiotics", BIOTECHNOLOGY RESEARCH INTERNATIONAL, vol. 1, no. 2, 1 January 2014 (2014-01-01), US, pages 13 - 19, XP055328529, ISSN: 2090-3138, DOI: 10.1016/ j.idairyj.2007.11.025
- JIN-WOO JEONG ET AL: "Anti-Inflammatory Effects of 3-(4'-Hydroxyl-3',5'-Dimethoxyphenyl) Propionic Acid, an Active Component of Korean Cabbage Kimchi, in Lipopolysaccharide-Stimulated BV2 Microglia", JOURNAL OF MEDICINAL FOOD, vol. 18, no. 6, 1 June 2015 (2015-06-01), US, pages 677 - 684, XP055328592, ISSN: 1096-620X, DOI: 10.1089/jmf.2014.3275
- IN HWA CHOI ET AL: "Kimchi, a Fermented Vegetable, Improves Serum Lipid Profiles in Healthy Young Adults: Randomized Clinical Trial", JOURNAL OF MEDICINAL FOOD, vol. 16, no. 3, 1 March 2013 (2013-03-01), US, pages 223 - 229, XP055328576, ISSN: 1096-620X, DOI: 10.1089/jmf.2012.2563
- G CHIANG ET AL: "Effects of Feeding Solid-state Fermented Rapeseed Meal on Performance, Nutrient Digestibility, Intestinal Ecology and Intestinal Morphology of Broiler Chickens", ASIAN-AUSTRALASIAN JOURNAL OF ANIMAL SCIENCES, vol. 23, no. 2, 1 February 2010 (2010-02-01), pages 263 - 271, XP055036607, DOI: 10.5713/ajas.2010.90145
- VIG A P ET AL: "Bio-protective effects of glucosinolates - A review", LWT- FOOD SCIENCE AND TECHNOLOGY, ACADEMIC PRESS, UNITED KINGDOM, vol. 42, no. 10, 1 December 2009 (2009-12-01), pages 1561 - 1572, XP026502411, ISSN: 0023-6438, [retrieved on 20090529], DOI: 10.1016/J.LWT.2009.05.023
- KIM, MEE-KYUNG; HONG, EUN-YOUNG; KIM, GUN-HEE;: "Change of Total Glucosinolates Level according to Processing Treatments in Chinese Cabbage (Brassica campestris L. ssp. Pekinensis) from Different Harvest Seasons", KOREAN JOURNAL OF HORTICULTURAL SCIENCE AND TECHNOLOGY, vol. 28, no. 4, 2010, pages 593 - 599, XP002765230
- VIG A P ET AL: "Beneficial effects of Rhizopus oligosporus fermentation on reduction of glucosinolates, fibre and phytic acid in rapeseed (Brassica napus) meal", BIORESOURCE TECHNOLOGY, ELSEVIER BV, GB, vol. 78, no. 3, 1 July 2001 (2001-07-01), pages 309 - 312, XP002346528, ISSN: 0960-8524, DOI: 10.1016/ S0960-8524(01)00030-X

Remarks:

The file contains technical information submitted after the application was filed and not included in this specification

(52) Cooperative Patent Classification (CPC): (Cont.)
A61P 25/00; A61P 29/00; A61P 31/04; A61P 37/02; A61P 37/08

C-Sets
A61K 35/20, A61K 2300/00;
A61K 35/744, A61K 2300/00;
A61K 35/747, A61K 2300/00;
A61K 36/31, A61K 2300/00

## Description

### Technical field of the invention

The present invention relates to the use of a substance comprising a combination of Brassica spp. and seaweed or algae; and one or more lactic acid bacterial strains showing nutritional and/or pharmacological effects.

### Background of the invention

In ancient times herbal medicine was the way, an often the only way, to fight diseases and infections in humans and animal, and it has been known that plants comprise many constituents having a positive nutritional and pharmacological effects. Despite this long term knowledge and search for isolating the active ingredients from plants, researchers seldom succeed because of the complexity and interplay between the individual components of the plants. Examples of potent extracts from plants that are well integrated in modern medical treatment can be found in drugs such as Morphine for treatment of severe pain, Digitalis for treatment of heart disease, Taxole for cancer treatment.

One of the components of interest and showing a positive nutritional and pharmacological effect is glucosinolates. The by-products obtained from glucosinolates are considered to have a protective effect against some cancer forms.

Glucosinolates are secondary plant metabolites that occur naturally in e.g. the plant *Brassicaceae.* Based on the amino acid origin of the side chain, glucosinolates are divided into aliphatic, aromatic, and indole glucosinolates, which are derived from methionine, phenylalanine, and tryptophan, respectively. The glucosinolates are natural components of e.g. many pungent plants such as mustard, cabbage, and horseradish. The pungency of those plants is due to mustard oils produced from glucosinolates when the plant material is chewed, cut, or otherwise damaged. These natural chemicals most likely contribute to plant defense against pests and diseases, but are also enjoyed in small amounts by humans and are believed to contribute to the health promoting properties of cruciferous vegetables.

Even there is an interest in the positive nutritional and pharmacological effects of the by-products obtained from glucosinolates, there is also a focus and interest in the industry to reduce or even avoid the presence of glucosinolates in the food or feed products since glucosinolates are considered in the prior art to have toxic effects in both humans and animals.

The European oil industry (FEDIOL) has in 2010 recommended their members to ensure that the rapeseed suppliers ensure and guaranties that the rapeseed products, and that certified rapeseed planting seeds used, has a glucosinolate level below 18 μmol/g. This being reduced from a previous level of 25 μmol/g.

Furthermore, a research group has previously described (in WO 2014/131422) a method for producing a compound being beneficial against *salmonella, E. Coli* and *lawsonia* infections in chickens. The idea behind was based on fermented feed product which had been fermented to such an extent that substantially no glucosinolates was remaining in the fermented feed. The feed product provided was substantially absent in glucosinolates, but rich in glucosinolate by-products, which was shown to be effective against *salmonella, E. Coli* and *lawsonia* infections in chickens.

### Summary of the invention

The inventors of the present invention surprisingly found a fermented plant material comprising high content of lactic acid bacterial strains and a high content of glucosinolates having nutritional and/or pharmacological effects.

The inventors of the present invention surprisingly found that a new method was developed for producing a novel fermented composition comprising a high content of lactic acid bacterial strains and a high content of glucosinolates and at the same time having nutritional and/or pharmacological effects.

Thus, one aspect of the invention relates to a substance for use as set out in the appended set of claims.

### Brief description of the figures

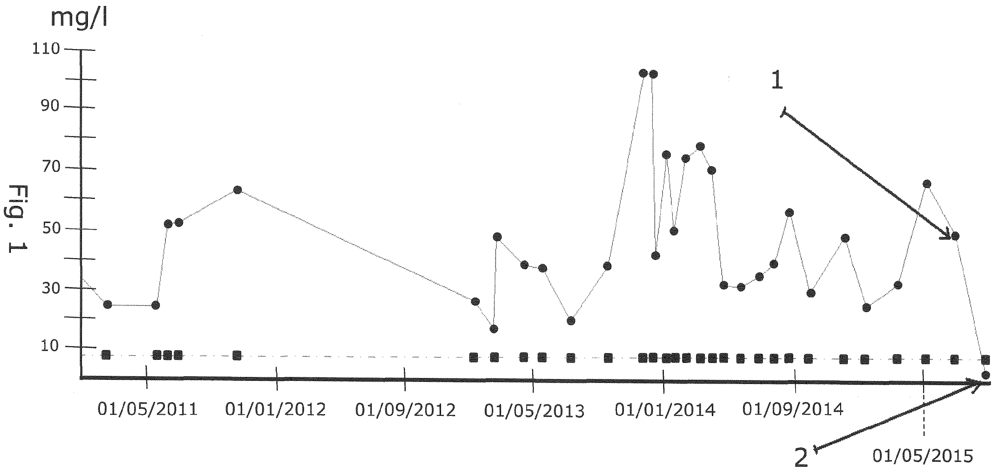

Figure 1 shows the effect, determined by the CRP level, of the fermented composition of the present invention on spondylarthritis/psoriatic arthritis in a patient. The effect demonstrated in figure 1 relates to a 58 years old male patient with HLA B27 antigen associated spondylarthritis/psoriatic arthritis. Treatment with disease modifying anti Rheumatic Drugs (DMARD) with a TNF alpha antibody and methotrexate in full dose for several years. Despite combination therapy with sDMARD and bDMARD it was not possible to normalize CRP before using an adjuvant in form of the fermented composition as described herein. Circles (●) indicate the CRP level taken from the patient. The squares (■) indicate the normalised level of the CRP in a healthy human. The treatment using the fermented composition according to the present invention was started at point 1 and after 17 days of daily administering the fermented composition the CRP level was normalised, at point 2.

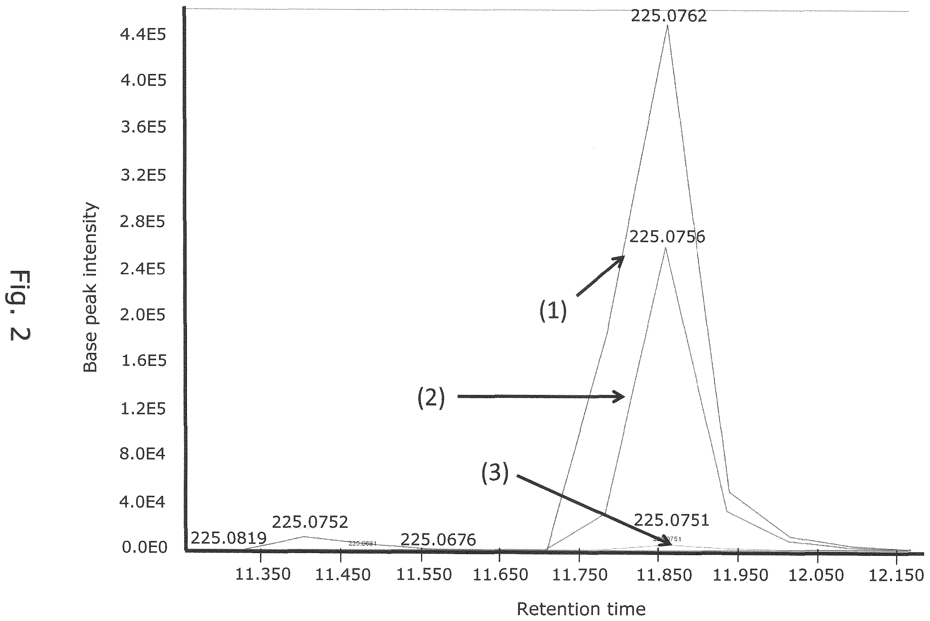

Figure 2 shows a chromatogram of the amount of HDMPPA (3-(4'-hydroxyl-3',5'-dimethoxyphenyl)propionic acid) in a fermented rapeseed/seaweed (1); fermented rapeseed (2); and non-fermented rapeseed (3). The amount is determined by Ultra High Pressure Liquid Chromatography-Quadruple Time of Flight Mass Spectroscopy (UPLC-QTOF-MS) by analysing an 80% methanol extract. From this chromatogram it is shown that fermented rapeseed/seaweed (1) has significant more HDMPPA relative to fermented rapeseed (2), which has significant more HDMPPA relative to non-fermented rapeseed (2).

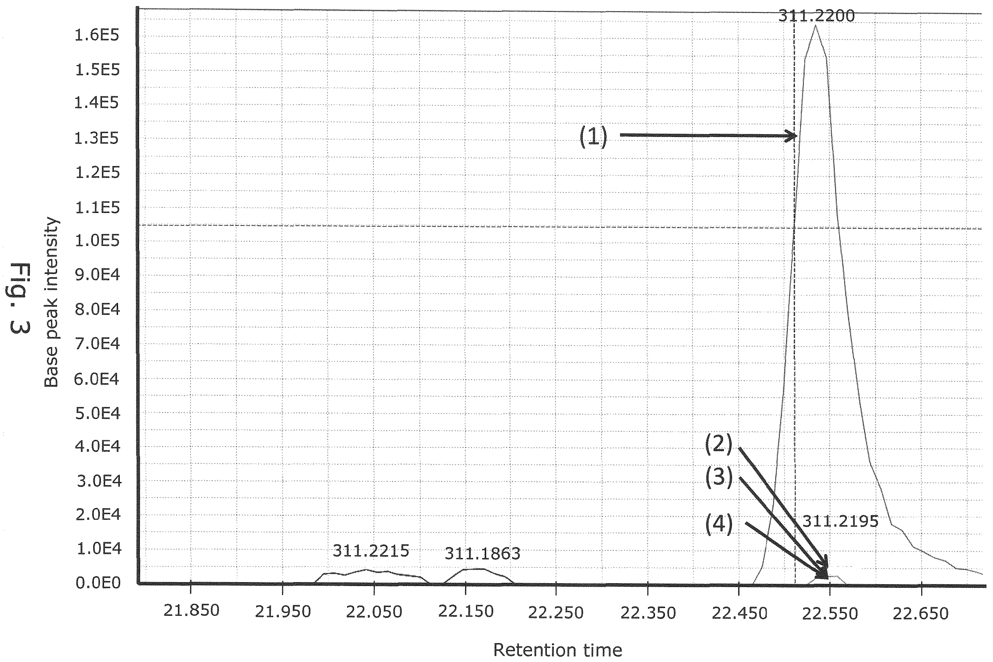

Figure 3 shows a chromatogram of a compound found in significant amounts in a fermented rapeseed/seaweed (1); whereas fermented rapeseed (2); non-fermented seaweed (3); and non-fermented rapeseed (4) (determined by Ultra High Pressure Liquid Chromatography-Quadruple Time of Flight Mass Spectroscopy (UPLC-QTOF-MS) by analysing an 80% methanol extract) shows no or insignificant amounts of the compound. In combination with further analysis (using Ultra High Pressure Liquid Chromatography-Quadruple Time of Flight Mass Spectroscopy (UPLC-QTOF-MS)) of molecular masses MS1 and MS2 of the fermented rapeseed/seaweed (1) suggested the compound found in the fermented rapeseed/seaweed (1); compared to the fermented rapeseed (2); non-fermented seaweed (3); and non-fermented rapeseed (4) was a fatty acid molecule, such as a modified fatty acid molecule.

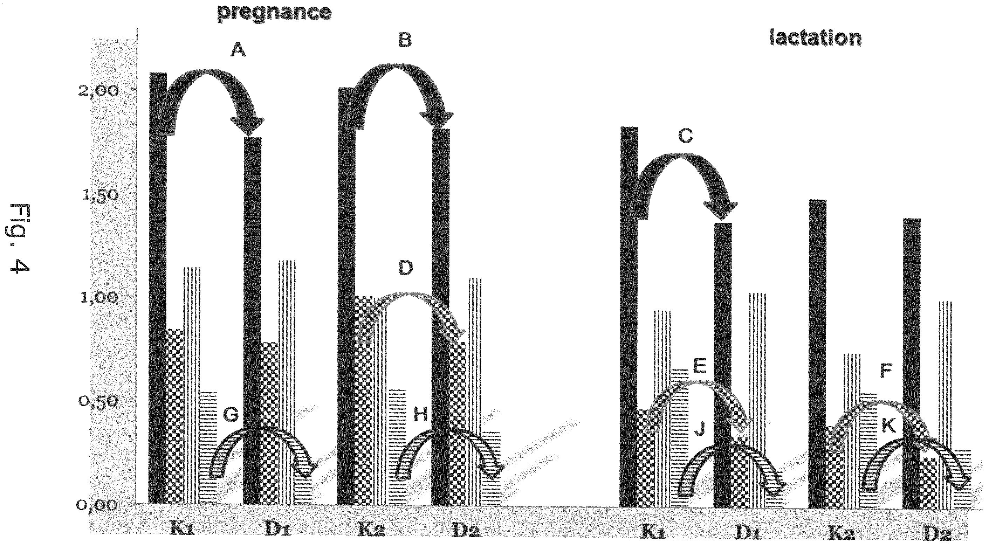

Figure 4 shows the effect of the fermented composition according to the present invention on cholesterol and liver damages. Two different breeds of sows were tested 1) the polish large white and 2) the polish landrace. The tests were made on both pregnant and lactating sows. K1 relates to polish large white sows fed normal feed without the fermented composition; K2 relates to polish landrace sows fed normal feed without the fermented composition; K1 relates to polish large white sows fed normal feed without the fermented composition; and K2 relates to polish landrace sows fed normal feed without the fermented composition. Solid bars and arrows indicate the concentration of total cholesterol and pregnant polish large white sows feed with the composition obtained in example 1 experienced a total cholesterol reduction (A) of 15%; whereas pregnant polish landrace sows experienced a total cholesterol reduction (B) of 10%; and lactating polish large white sows experienced a total cholesterol reduction (C) of 25%. Horizontally hatched bars and arrows indicate LDL cholesterol. Pregnant polish large white sows experienced a LDL cholesterol reduction (G) of 58%, pregnant polish landrace sows only experienced a LDL cholesterol reduction (H) of 36%; lactating polish large white sows, which experienced a LDL cholesterol reduction (J) of 72%; and lactating polish landrace sows experienced a LDL cholesterol reduction (K) of 49%. Dashed bars and arrows indicates the concentration of triglycerides. Here, pregnant polish landrace sows experienced a triglycerides reduction (D) of 21%; lactating polish large white sows experienced a triglycerides reduction (E) of 28%, and lactating polish landrace sows experienced a triglycerides reduction (F) of 38%.

**Detailed description of the invention**

Over the years, the interest in probiotics and prebiotics has kept increasing and the numbers of applications for which they are used have continuously enlarged and the nutritional and/or pharmacological effects are expanding. In this respect the inventors of the present invention surprisingly found that a combined fermented composition comprising probiotics and prebiotics show unexpected and improved nutritional and pharmacological effects.

Prebiotics are well known to the skilled person and refer to chemicals that induce the growth or activity of microorganisms (e.g., bacteria and fungi) that, when consumed by a human or an animal, contribute to the well-being of the host. The most common example of using prebiotics is in the gastrointestinal tract, where prebiotics can alter the composition of micro-organisms in the gut microflora.

Probiotics are also well known to the skilled person and are live microorganisms that, when administered to a human or an animal, are believed to provide health benefits.

A process for preparing a fermented composition, in particular a fermented composition comprising a prebiotic and a probiotic, may be described, the method comprises the steps of:

(i) providing a plant material;
(ii) providing one or more lactic acid bacteria strain(s);
(iii) subjecting the plant material to fermentation by combining the plant material provided in step a) with the one or more lactic acid bacterial strain(s) provided in step b),
(iv) continuing the fermentation until the fermented composition is provided having a pH value below 5.0 and having:

(a) a total glucosinolate content of at least 2 μmol/g or more on a dry-matter basis, and/or
(b) at least 10% (w/w) of the glucosinolates naturally present in the at least one plant material.

In a preferred embodiment of the present invention the fermented composition comprises one or more probiotic bacterial strain(s). Preferably, different strain(s) of probiotic bacteria may be used according to the present invention.

When the plant material is seaweed or algae the method for preparing the fermented composition may be as described herein or as described in WO 2014/206419.

In an embodiment of the present invention one or more probiotic bacterial may be lactic acid-producing bacteria, Lactic acid bacteria. Because of the activity of lactic acid bacteria they are often linked with food fermentations as acidification inhibits the growth of spoilage agents. Proteinaceous bacteriocins, toxins produced by bacteria to inhibit the growth of similar or closely related bacterial strain(s), are produced by several lactic acid bacteria strains and provide an additional hurdle for spoilage and pathogenic microorganisms.

Furthermore, lactic acid bacteria may produce lactic acid and other metabolic products which contribute to the organoleptic, textural, nutritional and pharmacological profile of the fermented composition.

The industrial importance of the lactic acid bacteria may be evidenced by their generally regarded as safe (GRAS) status, due to their ubiquitous appearance in food and their contribution to the healthy microflora of human mucosal surfaces. The genera that comprise the lactic acid bacteria, and which may be used in the present invention, are Lactobacillus, Leuconostoc, Pediococcus, Lactococcus, and Streptococcus, Aerococcus, Carnobacterium, Enterococcus, Oenococcus, Teragenococcus, Vagococcus, and Weisella; these genera belong to the order Lactobacillales.

In the present invention, the one or more lactic acid bacterial strain(s) provided in step (iii) and used for fermentation may be selected from lactic acid bacteria selected from the group consisting of Lactobacillus, Leuconostoc, Pediococcus, Lactococcus, and Streptococcus, Aerococcus, Carnobacterium, Enterococcus, Oenococcus, Teragenococcus, Vagococcus, and Weisella. Preferably, the one or more lactic acid bacterial strain(s) provided in step (iii) and used for fermentation may be selected from lactic acid bacteria selected from the group consisting of lactic acid bacteria of the genus Enterococcus, Lactobacillus, Pediococcus or Lactococcus, or combinations thereof.

In an embodiment of the present invention, the one or more lactic acid bacterial strain(s) provided in step (iii) and used for fermentation may be selected from the group consisting of one or more Enterococcus spp., Lactobacillus spp., Lactococcus spp., Pediococcus spp., and a combination hereof. Preferably, the one or more lactic acid bacterial strain provided in step (iii) and used for fermentation may be selected from the group consisting of one or more one Enterococcus faecium, Lactobacillus rhamnosus, Lactobacillus plantarum, Pediococcus acidililactili, Pediococcus pentosaceus, Lactococcus Lactis, Lactococcus Cremoris, Lactococcus Diacetylactis, Leuconostoc Cremoris and a combination hereof.

In a further embodiment, the one or more lactic acid bacterial strain(s) provided in step (iii) and used for fermentation comprise Lactobacillus plantarum, Enterococcus faecium and/or Lactobacillus rhamnosus.

In still another embodiment, the one or more lactic acid bacterial strain(s) provided in step (iii) and used for fermentation comprise one or more of Enterococcus faecium MCIMB 30122, Lactobacillus rhamnosus NCIMB 30121, Pediococcus pentosaceus HTS (LMG P-22549), Pendiococcus acidilactici NCIMB 30086 and/or Lactobacillus plantarum LSI (NCIMB 30083) or a combination hereof.

In order to increase productivity and effectivity two or more lactic acid bacterial strains may be provided, such as three or more lactic acid bacterial strains, e.g. four or more lactic acid bacterial strains, such as 7 or more lactic acid bacterial strains, e.g. 10 or more lactic acid bacterial strains, such as 15 or more lactic acid bacterial strains, e.g. 20 or more lactic acid bacterial strains, such as 25 or more lactic acid bacterial strains, e.g. 30 or more lactic acid bacterial strains, such as 35 or more lactic acid bacterial strains, e.g. 40 or more lactic acid bacterial strains.

In an embodiment of the present invention a starter culture or an inoculum may be provided comprising one or more lactic acid bacterial strain(s) as defined herein.

The term "inoculum" relates to a source material, such as the one or more lactic acid bacterial strain(s), used for the inoculation of a new culture. The inoculum may be employed to prime a process of interest.

"Inoculation" refers to the placement of a microorganism(s) (e.g. one or more lactic acid bacterial strain(s)) that will grow when implanted in a culture medium such as a fermentation tank comprising media to be fermented, e.g. a plant material.

A primary inoculum may be provided and refers to the generation of an initial inoculum in a series of repeated similar of essentially identical inoculation process, for example one or more repetitions of a fermentation process. An aliquot of the product of the formation process may be used to inoculate a new process of fermentation. Thus, the inoculation may be a fermented feed product which comprises viable lactic acid producing bacteria in sufficient amount to prime a lactic acid fermentation process of a another feed product, or similar feed, to be fermented, e.g. a plant material.

The inoculum according to the present invention may be a in a liquid form, dry form, or essentially dry form. The moisture content of the inoculum may be adjusted in order to optimize the fermentation process. In one embodiment the inoculum may be provided as essentially pure viable bacteria (such as bacteria in freeze dried form) or bacteria suspended in a suitable media prior to the application (such as a water, buffer or a growth media).

The proportion of the inoculums added to the plant material may vary. In case it is considered that the load of undesirable microbes are significant in the plant material or the fermentation system, the proportion of the inoculum in the fermentation mixture (inoculum + plant material + additional water) may be increased to insure that the fermentation is directed by the microbes (e.g. lactic acid bacteria) of the inoculums. Thus, the inoculum may be provided with a concentration of lactic acid bacteria in the inoculum sufficient to outgrow other (non-lactic acid bacteria, yeast or moulds present in the plant material.

Accordingly, in one embodiment of the invention, the proportion of the inoculums in the combination of the plant material and the one or more lactic acid material as defined in step (iii) is in the range of 0.1 to 99.9 vol-%; such as 1 to 99 vol-%; e.g. 5 to 70 vol-%; such as 10 to 50 vol-%; e.g. 25 to 35 vol-%; such as 0.1 to 10 vol-%; e.g. 0.5 to 5 vol-%; such as 1 to 2.5 vol-%; or around 1 to 2 vol-%.

The fermentation process according to the present invention, may preferably be essentially a homofermentative process. "Essentially homofermentative" means, that the predominant bacterial flora driving the fermentation is homofermentative. In the present context the term "essentially homofermentative" relates to a fermentation process where, 60% or more of the bacteria are homofermentative, such as 70% or more of the bacteria are homofermentative, e.g. 80% or more of the bacteria are homofermentative, such as 85% or more of the bacteria are homofermentative, e.g. 90% or more of the bacteria are homofermentative, such as 95% or more of the bacteria are homofermentative, e.g. 99% or more of the bacteria are homofermentative.

In an embodiment of the present invention the fermentation is essentially a homofermentation, such as a homolactic fermentation.

The term "homolactic fermentation" when used according to the present invention indicates that the major fermentation product is lactic acid, and the levels of acetic acid and ethanol are either below taste threshold, around taste threshold or slightly above taste threshold. Preferably, "essentially homofermentative" indicates a ratio of lactic acid to acetic acid or lactic acid to ethanol in (mM/mM) of more than 1:1, such as 2:1 or more, e.g. 10:1 or more, such as 20:1 or more, e.g. 50:1 or more, or such as 100:1 or more.

In the present context the term "essentially heterofermentative" means, that the predominant bacterial flora driving the fermentation is heterofermentative. In the present context the term "essentially heterofermentative" relates to a fermentation process where, 60% or more of the bacteria are heterofermentative, such as 70% or more of the bacteria are heterofermentative, e.g. 80% or more of the bacteria are heterofermentative, such as 85% or more of the bacteria are heterofermentative, e.g. 90% or more of the bacteria are heterofermentative, such as 95% or more of the bacteria are heterofermentative, e.g. 99% or more of the bacteria are heterofermentative.

The fermentation may be continued for 10 days or less, such as 9 days or less, e.g. 8 days or less, such as 7 days or less, e.g. 6 days or less, such as 5 days or less, e.g. 4 days or less, such as 3 days or less, e.g. 2 days or less, such as 36 hours or less, e.g. 24 hours or less.

Even the fermentation should be continued as quickly as possible, the inventors also found that a certain time of fermentation may be required to provide the desired nutritional and/or pharmacological effects of the fermented composition. Hence, the fermentation should be continued for more than 8 hours, such as more than 10 hours, e.g. more than 12 hours, such as more than 15 hours, e.g. more than 17 hours, such as more than 20 hours, e.g. more than 24 hours, such as in the range of 24 hours to 10 days, e.g. in the range of 20 hours to 8 days, such as in the range of 15 hours to 6 days, e.g. in the range of 12 hours to 4 days, such as in the range of 10 hours to 2 days, e.g. in the range of 8 hours to 36 hours, such as in the range of 8 hours to 24 hours.

Controlling the temperature may be one of the best ways to improve the quality and the effects of the fermented composition. The fermentation may be performed at a temperature below 50°C, such as below 47°C, e.g. below 45°C, such as below 43°C, e.g. in the range 15-45°C, such as 18-43°C, such as 25-40°C, such as 30-40°C, such as 15-20°C or such as 40-45°C.

The fermentation process may preferably involve a temperature gradient, said temperature gradient involves 3 stages; a starting temperature, a temperature increases and a steady state fermentation temperature.

In an embodiment of the present invention the starting temperature may be in the range of 18-30°C, such as in the range of 20-28°C, e.g. in the range of 22-26°C, such as in the range of 24-25°C.

The temperature increase of the fermentation process may preferably be a slow temperature increase from the starting temperature to the steady state fermentation temperature. In an embodiment of the present invention the temperature increase is provided without addition of heat. In the present context the term "without addition of heat" relates to a fermentation temperature increase wherein the heat provided is produced by the fermentation itself without the use of electrical, mechanical or fuel based heat.

The steady state fermentation temperature may be below 50°C, such as below 47°C, e.g. below 45°C, such as below 43°C, e.g. in the range 20-45°C, such as in the range 22-43°C, such as in the range 25-40°C, such as in the range 30-40°C, such as in the range 40-45°C.

The fermentation may be a one-step fermentation of the plant material.

In the present context, the term "one-step fermentation" relates to a fermentation process wherein the same type of plant material is subjected to the same fermentation conditions, or substantially the same fermentation conditions. Hence, the term "one-step fermentation", exclude the option of taking out a part of the plant material during fermentation, leaving the remaining plant material to be further fermented and followed by mixing the part which was taken out with the further fermented material. Preferably, the term "one-step fermentation" also includes a temperature increase provided without addition of heat.

In a further embodiment of the present invention, the fermented composition does not involve subsequent supplementation of plant material and/or one or more lactic acid bacterial strain(s) to the fermented composition.

The moisture content of the plant material to be fermented may be another relevant parameter to control in order to control the fermentation process and the resulting fermented composition. Thus, in an embodiment of the present invention the moisture content during the fermentation may be in the range of 20-60% (w/w), such as in the range of 30-50% (w/w), e.g. preferably in the range of 35-45% (w/w).

The fermentation may be continued until the fermented composition has a pH below pH 6.5, such as below pH 6.0, e.g. below pH 5.5, such as below pH 5.0, e.g. in the range of pH 3.0-6.5, such as in the range of pH 3.0-6.0, e.g. in the range of pH 3.1-5.5, such as in the range of pH 5.0-3.2, such as in the range 3.3- 4-2, such as 3.4-4.0, such as 3.5-3.8, such as 3.7-4.2, such as 3.7-4.0, or such as 3.8-4.2.

The fermented composition provided in step iv) may have a lactic acid concentration of at least 50 mM, such as at least 100 mM, such as 100-1000 mM, such as 100-500 mM, such as 100-300 mM, such as 100-200 mM, such as 150-500 mM, such as 200-500 mM or such as 300-500, mM lactic acid.

Since it is believed that the probiotic effect of the one or more lactic acid bacterial strain(s) peaks after 6 hours to 10 days, such as 12 hours to 9 days, e.g. 18 hours to 8 days, such as 24 hours to 7 days, e.g. 2-6 days, such as after 3-5 days, e.g. after 4-5 days, such as 6 hours to 4 days, such as 6 hours to 3 days, e.g. 6 hours to 2 days, such as 6 hours to 24 hours, e.g. 6 hours to 18 hours, such as 6 hours to 12 hours the fermentation process may be stopped within this time period. After the fermentation is stopped the fermented composition may be dried in order to prolong the viability (CFU) of the one or more lactic acid bacterial strain(s).

The fermented composition according the present invention may be a liquid, a slurry, or a dry powder.

The process may further comprises a step of reducing the moisture content from the moisture content obtained from the fermentation process to a fermented composition moisture content. The present invention the composition may be subjected to drying.

Any moisture reducing methods may be used which are sensitive to the pre- and probiotic components of the fermented composition in order to maintain the activity of the components in the fermented composition and that ensure high viability of the lactic acid bacteria present in the fermented composition. The method to reduce to moisture content as described in WO 2013/029632 may be preferred .

In order to improve the fermentation process, the plant material may be pre-treated before combining the plant material with the one or more lactic acid bacterial strain(s) as described in step (iii).

Such pre-treatment may involve grinding, cutting, chopping, slicing, and/or fractionizing the plant material.

The plant material provided in step (i) may have, optionally after a pre-treatment, an average diameter of 5 mm or less, such as an average diameter of 4 mm or less, such as an average diameter of 3 mm or less, such as an average diameter of 2 mm or less, such as an average diameter of 1 mm or less, such as an average diameter in the range 25 $\mu$m to 5 mm, such as 0.1 mm to 4 mm, such as an average diameter in the range of 0.5 mm to 2.5 mm, such as an average diameter in the range 0.5 mm to 2 mm.

Determination of the average diameter of the plant material may e.g. be performed by microscopic inspection, laser diffraction or sieving.

In an embodiment of the present invention the plant material may have a natural glucosinolate content of at least 3 $\mu$mol/g on a dry matter basis, such as at least 4 $\mu$mol/g on a dry matter basis, e.g. at least 5 $\mu$mol/g on a dry matter basis, such as at least 7.5 $\mu$mol/g on a dry matter basis, e.g. at least 10 $\mu$mol/g on a dry matter basis, such as at least 12.5 $\mu$mol/g on a dry matter basis, e.g. at least 15 $\mu$mol/g on a dry matter basis, such as at least 17.5 $\mu$mol/g on a dry matter basis, e.g. at least 20 $\mu$mol/g on a dry matter basis, such as at least 25 $\mu$mol/g on a dry matter basis, e.g. at least 30 $\mu$mol/g on a dry matter basis, such as at least 40 $\mu$mol/g on a dry matter basis, e.g. at least 50 $\mu$mol/g on a dry matter basis, such as at least 60 $\mu$mol/g on a dry matter basis, e.g. at least 70 $\mu$mol/g on a dry matter basis, such as at least 80 $\mu$mol/g on a dry matter basis, e.g. at least 90 $\mu$mol/g on a dry matter basis, such as at least 100 $\mu$mol/g on a dry matter basis, e.g. at least 125 $\mu$mol/g on a dry matter basis, such as at least 150 $\mu$mol/g on a dry matter basis.

In the present context, the term "natural content" relates to a determined/analysed content of glucosinolates in the plant material before fermentation. If no determination or analysis of the plant material before has been made or can be made the "natural content" relates to the natural content to be found in the literature for the specific plant material.

In the present context, the term "plant material" relates to organisms (such as plants, seaweed or algae) capable of performing photosynthesis.

In an embodiment of the present invention, the plant material may be Brassica spp. and brown algae.

In an embodiment of the present invention, the *Brassica spp.* may preferably be selected from one or more of rape species; cruciferous vegetables; cabbage species; and/or mustard species. Preferably, the *Brassica spp.* may preferably be selected from one or more of rape species. Preferably, the rape species is a rapeseed product, such as rapeseed meal, or rapeseed cake, preferably rapeseed cake.

In another embodiment of the present invention, the *Brassica spp.* may be selected from one or more species such as *Brassica napus; Brassica oleracea;* Brassica campestris; and/or *Brassica rapa.*

In a further embodiment of the present invention, the seaweed and/or algae may be selected from one or more of brown algae, such as kelps, *Laminaria saccharina, Laminaria digitata,* and/or *Laminaria hyperborean.*

In an even further embodiment of the present invention, the plant material comprises a combination of *Brassica spp.,* in particular *Brassica napus;* or Brassica campestris, and brown algae.

In the event the plant material comprises a combination of *Brassica spp.,* in particular *Brassica napus;* or Brassica campestris, and brown algae, the brown algae may preferably be subjected to the pre-treatment to an extent that result in an average diameter which is at most 75% of the average diameter of the *Brassica spp.,* such as at most 50% of the average diameter.

The *Brassica spp.* provided in step (i) may have, optionally after a pre-treatment, an average diameter of 3 mm or less, such as an average diameter of 2 mm or less, such as an average diameter of 1 mm or less, such as an average diameter in the range 25 $\mu$m to 3 mm, such as 0.1 mm to 2.5 mm, such as an average diameter in the range of 0.5 mm to 2.25 mm, such as an average diameter in the range 1.0 mm to 2 mm.

The brown algae provided in step (i) may have, optionally after a pre-treatment, an average diameter of 2 mm or less, such as an average diameter of 1.5 mm or less, such as an average diameter of 1 mm or less, such as an average diameter in the range 25 $\mu$m to 2 mm, such as 0.1 mm to 1.5 mm, such as an average diameter in the range of 0.5 mm to 1.25 mm, such as an average diameter in the range 0.75 mm to 1 mm.

In yet an embodiment of the present invention, the ratio between *Brassica spp.* and brown algae is at least 1:1, such as at least 1:2, e.g. at least 1:3, such as at least 1:4, e.g. at least 1:5, such as at least 1:6, e.g. at least 1:7, such as at least 1:8, e.g. at least 1:9, such as at least 1:10.

The plant material may be provided in the form of seeds, press cake, meal or other residues of industrial utilization of said plant material. Preferably, the plant material may be provided in the form of cake or meal, even more preferably, the plant material may be provided in the form of cake.

The plant material provided in step a) and the one or more lactic acid bacterial strain(s) provided in step b) may be further supplemented with one or more further ingredient selected from the group consisting of cereals, (e.g. wheat, barley, rye, rice, maize (cob maize silage (CCM) or ripe), triticale, oat); vegetables (e.g. potatoes, beans, peas, maize, soy); and/or carbohydrate ingredient (such as whey, curd, skim milk and the like).

When the combination of the plant material provided in step a) and the one or more lactic acid bacterial strain(s) provided in step b) may be further supplemented with cereals, preferably wheat, the cereal constitutes 1-15% (w/w) of the composition, such as 5-10% (w/w) of the composition.

When the combination of the plant material provided in step a) and the one or more lactic acid bacterial strain(s) provided in step b) may be further supplemented with vegetables, preferably starch containing vegetables such as potatoes, the vegetables constitutes 0.1-5% (w/w) of the composition, such as 0.5-2% (w/w) of the composition

When the combination of the plant material provided in step a) and the one or more lactic acid bacterial strain(s) provided in step b) may be further supplemented with carbohydrate ingredient, such as whey, the carbohydrate ingredient constitutes 0.1-5% (w/w) of the composition, such as 0.5-2% (w/w) of the composition.

The fermentation process may preferably be an anaerobic fermentation process or a substantially anaerobic fermentation process.

In the context of the present invention the term "substantially anaerobic fermentation" relates to fermentation where no oxygen or other respiratory metabolism or oxidative phosphorylation is being established. Hence, the term "substantially anaerobic fermentation" means that oxygen (or other electron acceptor compounds) is not systematically added to the fermentation. The oxygen naturally present and included when setting up the fermentation process may be accepted.

The process was shown to be suitable for producing a novel fermented plant material comprising a high content of lactic acid bacterial strains and a high content of glucosinolates and showing nutritional and/or pharmacological effects.

In an embodiment of the present invention the fermented composition may have a glucosinolate content of at least 3 $\mu$mol/g on a dry matter basis, such as at least 4 $\mu$mol/g on a dry matter basis, e.g. at least 5 $\mu$mol/g on a dry matter basis, such as at least 7.5 $\mu$mol/g on a dry matter basis, e.g. at least 10 $\mu$mol/g on a dry matter basis, such as at least 12.5 $\mu$mol/g on a dry matter basis, e.g. at least 15 $\mu$mol/g on a dry matter basis, such as at least 17.5 $\mu$mol/g on a dry matter basis, e.g. at least 20 $\mu$mol/g on a dry matter basis, such as at least 25 $\mu$mol/g on a dry matter basis, e.g. at least 30 $\mu$mol/g on a dry matter basis, such as at least 40 $\mu$mol/g on a dry matter basis, e.g. at least 50 $\mu$mol/g on a dry matter basis, such as at least 60 $\mu$mol/g on a dry matter basis, e.g. at least 70 $\mu$mol/g on a dry matter basis, such as at least 80 $\mu$mol/g on a dry matter basis, e.g. at least 90 $\mu$mol/g on a dry matter basis, such as at least 100 $\mu$mol/g on a dry matter basis.

In an embodiment of the present invention the content of glucosinolates present in the fermented composition may be at least 10% (w/w) of the glucosinolates naturally present in the at least one plant material, such as at least 10% (w/w), e.g. at least 20% (w/w), such as at least 30% (w/w), e.g. at least 40% (w/w), such as at least 50% (w/w), e.g. at least 60% (w/w), such as at least 65% (w/w), e.g. at least 70% (w/w), such as at least 75% (w/w), e.g. at least 80% (w/w).

In yet an embodiment of the present invention the content of glucosinolates present in the fermented composition may be in the range of 10-80% (w/w) of the glucosinolates naturally present in the at least one plant material, such as in the range of 20-75% (w/w), e.g. in the range of 30-70% (w/w), such as in the range of 40-60% (w/w), e.g. in the range of 45-55% (w/w).

In the present context, the term "glucosinolates naturally present in the at least one plant material" relates to a determined/analysed content of glucosinolates in the plant material to be fermented - before fermentation. If no determination or analysis of the plant material before fermentation has been made or can be made, the "glucosinolates naturally present in the at least one plant material" relates to the amount of glucosinolates to be found in the literature for the specific plant material.

The glucosinolate content may be determined by high-performance liquid chromatography (HPLC), which is an analytical tool well known to the person skilled in the art. The determination of glucosinolates may be done in accordance with Commission Regulation (EEC) No 1864/90 of 29 June 1990, where the glucosinolates present in a plant material is extracted in a methanol/water solution, then purification and enzymatic desulphation on ion exchange resins followed by determination using reversed-phase high-performance liquid chromatography with elution gradient and UV detection.

In a preferred embodiment of the present invention the fermented composition comprises at least one plant material selected from the group consisting of rape species; cruciferous vegetables; mustard species, and/or seaweed, preferably rape species or the combination of rape species and seaweed; and one or more lactic acid bacteria, wherein the composition comprises at least 2 mg HDMPPA (3-(4'-hydroxyl-3',5'-dimethoxyphenyl)propionic acid) per 100 g fermented composition, on a dry matter basis, such as at least 3 mg/100 g, e.g. at least 4 mg/100 g, such as at least 5 mg/100 g, e.g. at least 7 mg/100 g, such as at least 10 mg/100 g, e.g. at least 15 mg/100 g, such as at least 20 mg/100 g, e.g. at least 30 mg/100 g, such as at least 40 mg/100 g, e.g. at least 50 mg/100 g, such as at least 60 mg/100 g, e.g. at least 70 mg/100 g, such as at least 80 mg/100 g.

HDMPPA (3-(4'-hydroxyl-3',5'-dimethoxyphenyl)propionic acid) is a compound originally found in kimchi as one of the major molecules. Kimchi is a fermented Korean vegetable side dish comprising cabbage red pepper, garlic ginger and lactic acid bacteria. HDMPPA is believed by researchers to be an active immune defence in the central nervous system, CNS (see Jin-Woo Jeong et al. 2015), to have anti-oxidative activity and prevent and/or treating atherosclerosis (Hyun Ju Kim, el al. 2007), to be lipid lowering, to have anti-tumor and anti-atherogenic effects, to have anti-viral effects against acute respiratory syndrome (Ye-Rang Yun et al. 2014). The amount of HDMPPA in kimchi is approximately 1 mg/100g kimchi.

The inventors of the present invention surprisingly found that the fermented plant material according to the present invention comprises significant more HDMPPA than the fermented product according to the present invention, relative to the amount of HDMPPA found in non-fermented plant material or in kimchi products described in the prior art.

In an embodiment of the present invention, the fermented composition comprises at least 2 mg HDMPPA (3-(4'-hydroxyl-3',5'-dimethoxyphenyl)propionic acid) per 100 g fermented composition, on a dry matter basis, such as at least 3 mg/100 g, e.g. at least 4 mg/100 g, such as at least 5 mg/100 g, e.g. at least 7 mg/100 g, such as at least 10 mg/100 g, e.g. at least 15 mg/100 g, such as at least 20 mg/100 g, e.g. at least 30 mg/100 g, such as at least 40 mg/100 g, e.g. at least 50 mg/100 g, such as at least 60 mg/100 g, e.g. at least 70 mg/100 g, such as at least 80 mg/100 g.

The significant increase in HDMPPA provided in the fermented composition according to the present invention may be provided by a unique combination of lactic acid bacteria used for the fermentation. Hence, the one or more lactic acid bacteria present in the composition may be selected from the group consisting of Pediococcus pentosaceus; Pendiococcus acidilactici; Lactobacillus plantarum; Lactobacillus rhamnosus; Enterococcus faecium and/or a combination thereof. Preferably, the lactic acid bacteria present in the composition may be Pediococcus pentosaceus; Pendiococcus acidilactici; Lactobacillus plantarum; Lactobacillus rhamnosus; and Enterococcus faecium.

In a preferred embodiment of the present invention, the fermented composition comprising a combination of Brassica spp, brown seaweed and one or more lactic acid bacteria, wherein the composition comprises at least 2 mg HDMPPA (3-(4'-hydroxyl-3',5'-dimethoxyphenyl)propionic acid) per 100 g fermented composition, on a dry matter basis, such as at least 3 mg/100 g, e.g. at least 4 mg/100 g, such as at least 5 mg/100 g, e.g. at least 7 mg/100 g, such as at least 10 mg/100 g, e.g. at least 15 mg/100 g, such as at least 20 mg/100 g, e.g. at least 30 mg/100 g, such as at least 40 mg/100 g, e.g. at least 50 mg/100 g, such as at least 60 mg/100 g, e.g. at least 70 mg/100 g, such as at least 80 mg/100 g and wherein one or more lactic acid bacteria present in the composition is selected from the group consisting of Pediococcus pentosaceus; Pendiococcus acidilactici; Lactobacillus plantarum; Lactobacillus rhamnosus; Enterococcus faecium and/or a combination thereof.

In an embodiment of the present invention, neither the composition nor the fermented composition comprises *Lactobacillus kimchi.*

In a preferred embodiment of the present invention the at least one plant material (when the composition comprises HDMPPA) is selected from the group consisting of rape species; cruciferous vegetables; mustard species, and/or seaweed, preferably rape species and/or seaweed. Preferably, the plant material is not a cabbage species. Even more preferably, the plant material is (when the composition comprises HDMPPA) a combination of rape species and seaweed.

In an embodiment of the present invention the fermented composition was analysed for HDMPPA and other relevant molecules by subjecting an 80% methanol extraction of the fermented composition to Ultra High Pressure Liquid Chromatography-Quadruple Time of Flight Mass Spectroscopy (UHPLC-Q-ToF-MS). Apart of a significant amount of HDPPA in the fermented plant material, an even more significant amount of a molecule was found in a fermented rapeseed/seaweed fraction (1); whereas fermented rapeseed (2); non-fermented seaweed (3); and non-fermented rapeseed (4), showed no or insignificant amounts of the molecule.

Mass Spectroscopy is a conventional technique used when a molecule found in nature or in complex media, and not synthetically produced, are to be determined or identified, based on the molecular mass of the molecule.

In order to define the molecule, the fermented composition was subjected to both MS1 analyses and MS2 analysis. In the present context the term "MS1 analysis" relates to the analysis of the intact molecule, with little fractionation, by mass spectroscopy, such as LC-QTOF-MS. In the present context the term "MS2 analysis" relates to the analysis of fragments of the molecule by mass spectroscopy, such as LC-QTOF-MS.

In an embodiment of the present invention the molecule has a molecular mass MS1 in the range of 280-340 Dalton, such as in the range of 300-320 Dalton, e.g. in the range of 305-315 Dalton, such as about 311 Dalton, e.g. about 311.2228 Dalton.

In yet an embodiment of the present invention, the molecule has a first molecular masses MS2 in the range of 210-230 Dalton, such as in the range of 215-225 Dalton, e.g. about 223 Dalton, such as about 223.1694 Dalton.

In a further embodiment of the present invention, the molecule has a second molecular masses MS2 in the range of 80-90 Dalton, such as in the range of 85-88 Dalton, e.g. about 87 Dalton, such as about 87.0436 Dalton.

In an even further embodiment of the present invention, the molecule has a third molecular masses MS2 in the range of 220-245 Dalton, such as in the range of 230-240 Dalton, e.g. about 235 Dalton, such as about 235.1666 Dalton.

In yet an embodiment of the present invention, the molecule has a fourth molecular masses MS2 in the range of 210-230 Dalton, such as in the range of 215-225 Dalton, e.g. about 224 Dalton, such as about 224.1725 Dalton.

In even further embodiment of the present invention, the molecule has a fifth molecular masses MS2 in the range of 265-285 Dalton, such as in the range of 270-280 Dalton, e.g. about 275 Dalton, such as about 275.2005 Dalton.

Preferably the molecule according to the present invention has a molecular mass MS1 in the range of 280-340 Dalton, such as in the range of 300-320 Dalton, e.g. in the range of 305-315 Dalton, such as about 311 Dalton, e.g. about 311.2228 Dalton; a first molecular masses MS2 in the range of 210-230 Dalton, such as in the range of 215-225 Dalton, e.g. about 223 Dalton, such as about 223.1694 Dalton; a second molecular masses MS2 in the range of 80-90 Dalton, such as in the range of 85-88 Dalton, e.g. about 87 Dalton, such as about 87.0436 Dalton; a third molecular masses MS2 in the range of 220-245 Dalton, such as in the range of 230-240 Dalton, e.g. about 235 Dalton, such as about 235.1666 Dalton; a fourth molecular masses MS2 in the range of 210-230 Dalton, such as in the range of 215-225 Dalton, e.g. about 224 Dalton, such as about 224.1725 Dalton; and a fifth molecular masses MS2 in the range of 265-285 Dalton, such as in the range of 270-280 Dalton, e.g. about 275 Dalton, such as about 275.2005 Dalton.

The various fractions of the MS2 molecular masses may be found in the fermented composition in the following relative concentrations/amounts first MS2 > second MS2 > third MS2 > fourth MS2 > fifth MS2.

Without being bound by any theory, the inventors of the present invention trust that the molecule according to the present invention is a fatty acid compound, preferably a modified fatty acid compound.

In an embodiment of the present invention the amount of the fatty acid compound may be increased relative to a non-fermented plant material.

Furthermore, the amount of the fatty acid compound may be increased relative to kimchi, such as a fermented composition comprising a cabbage species, such as Korean cabbage, or a fermented plant material mainly using *Lactobacillus kimchi.*

In the present context the term "mainly using" relates to a concentration of more than 50% of the total content of lactic acid bacteria in the composition or in the fermented composition being *Lactobacillus kimchi.*

The inventors surprisingly found that the molecule may be present in at least 2 times the concentration/amount found in non-fermented plant material, such as at least 5 times, e.g. at least 10 times, such as at least 15 times. It was surprisingly found that the molecule may be present in at least 2 times the concentration/amount found in kimchi, such as at least 5 times, e.g. at least 10 times, such as at least 15 times.

In an embodiment of the present invention, the molecule, e.g. the fatty acid compound, is absent in a non-fermented plant material, such as absent in fermented rapeseed, or in fermented seaweed.

The fermented composition according to the present invention may comprise the same one or more lactic acid bacterial strain(s) as provided in step (ii).

In an embodiment of the present invention at least one of the one or more lactic acid bacterial strain(s) as provided in step (ii) are not present in the fermented composition, such as at least two, e.g. at least 3, such as at least 4, e.g. at least 5, such as at least 10, e.g. at least 15.

In another embodiment of the present invention the fermented composition comprises one or more lactic acid bacterial strains, e.g. two or more lactic acid bacterial strains, such as three or more bacterial strains, e.g. four or more bacterial strains, such as 7 or more bacterial strains, e.g. 10 or more bacterial strains, such as 15 or more bacterial strains, e.g. 20 or more bacterial strains, such as 25 or more bacterial strains, e.g. 30 or more bacterial strains, such as 35 or more bacterial strains, e.g. 40 or more bacterial strains.

In a further embodiment of the present invention the one or more lactic acid bacterial strain(s) present in the fermented composition may be selected from the group consisting of *Lactobacillus, Leuconostoc, Pediococcus, Lactococcus, Streptococcus, Aerococcus, Carnobacterium, Enterococcus, Oenococcus, Teragenococcus,* Vagococcus, and *Weisella.* Preferably, the one or more lactic acid bacterial strain(s) present in the fermented composition are preferably lactic acid bacteria of the genus *Enterococcus, Lactobacillus, Pediococcus, Lactococcus,* or a combination thereof.

In an even further embodiment of the present invention the one or more lactic acid bacteria stain(s) may be selected from the group consisting of one or more *Enterococcus spp., Lactobacillus spp., Lactococcus spp., Pediococcus spp.,* and a combination hereof. Preferably, the one or more lactic acid bacterial strain(s) is/are selected from the group consisting of one or more of *Enterococcus faecium, Lactobacillus rhamnosus, Lactobacillus plantarum, Pediococcus acidililactili, Pediococcus pentosaceus, Lactococcus Lactis, Lactococcus Cremoris, Lactococcus Diacetylactis, Leuconostoc Cremoris* and a combination hereof.

In yet a further embodiment of the present invention, the main lactic acid bacterial strain(s) present in the composition may be *Pediococcus pentosaceus; Pendiococcus acidilactici; Lactobacillus plantarum; Lactobacillus rhamnosus;* or *Enterococcus faecium.* Preferably, the main lactic acid bacteria present in the composition may be *Lactobacillus plantarum.*

In another embodiment of the present invention the one or more lactic acid bacteria stain(s) may be selected from the group consisting of one or more of *Enterococcus faecium* MCIMB 30122, *Lactobacillus rhamnosus* NCIMB 30121, *Pediococcus pentosaceus* HTS (LMG P-22549), *Pendiococcus acidilactici* NCIMB 30086 and/or *Lactobacillus plantarum* LSI (NCIMB 30083).

In order to provide the desired effects, the fermented composition should have a high content of viable lactic acid bacteria. In an embodiment of the present invention the fermented composition comprises one or more lactic acid bacterial strain(s) in a total amount in the range of $10^5$-$10^{12}$ CFU per gram, such as in the range of $10^6$-$10^{12}$ CFU per gram, e.g. in the range of $10^7$-$10^{11}$ CFU per gram, such as in the range of $10^8$-$10^{11}$ CFU per gram, e.g. in the range of $10^9$-$10^{10}$ CFU per gram.

In order to improve the fermentation process and the effect of the fermented composition the fermented composition may comprise fractionized plant material. The plant material may be fractionized by grinding, cutting, chopping, slicing, and/or fractionizing providing a fractionized plant material.

The fermented composition according to the present invention may comprise a fractionized plant material having an average diameter of 5 mm or less, such as an average diameter of 4 mm or less, such as an average diameter of 3 mm or less, such as an average diameter of 2 mm or less, such as an average diameter of 1 mm or less, such as an average diameter in the range 25 $\mu$m to 5 mm, such as 0.1 mm to 4 mm, such as an average diameter in the range of 0.5 mm to 2.5 mm, such as an average diameter in the range 0.5 mm to 2 mm.

In an embodiment of the present invention the fermented composition may comprise a plant material comprising a combination of *Brassica spp.,* in particular *Brassica napus;* or Brassica campestris, and brown algae.

Preferably the brown algae present in the fermented composition may have an average diameter which is at most 75% of the average diameter of the *Brassica spp.,* such as at most 50% of the average diameter.

In an embodiment of the present invention, the *Brassica spp.,* when present in the fermented composition, has an average diameter of 3 mm or less, such as an average diameter of 2 mm or less, such as an average diameter of 1 mm or less, such as an average diameter in the range 25 $\mu$m to 3 mm, such as 0.1 mm to 2.5 mm, such as an average diameter in the range of 0.5 mm to 2.25 mm, such as an average diameter in the range 1.0 mm to 2 mm.

In another embodiment of the present invention, the brown algae may have an average diameter of 2 mm or less, such as an average diameter of 1.5 mm or less, such as an average diameter of 1 mm or less, such as an average diameter in the range 25 $\mu$m to 2 mm, such as 0.1 mm to 1.5 mm, such as an average diameter in the range of 0.5 mm to 1.25 mm, such as an average diameter in the range 0.75 mm to 1 mm.

When the fermented composition comprises a combination of *Brassica spp.,* in particular *Brassica napus;* or Brassica campestris, and brown algae the ratio between *Brassica spp.* and brown algae may be at least 1:1, such as at least 1:2, e.g. at least 1:3, such as at least 1:4, e.g. at least 1:5, such as at least 1:6, e.g. at least 1:7, such as at least 1:8, e.g. at least 1:9, such as at least 1:10.

In an embodiment of the present invention the content of the plant material in the fermented composition is at least 50% plant material, such as at least 55% plant material, e.g. at least 60% plant material, such as at least 65% plant material, e.g. at least 70% plant material, such as at least 75% plant material, e.g. at least 80% plant material, such as at least 85% plant material, e.g. at least 90% plant material, such as at least 95% plant material.

The fermented composition may be provided in many forms. Preferably, the fermented composition may be a liquid, a slurry, or a dry powder.

In an embodiment of the present invention a fermented composition is provided, which has a high content of lactic acid, high content of glucosinolates, and high pre- and probiotic activity. In accordance with the present invention the prebiotic may be the fermented plant material defined herein. In accordance with the present invention the probiotic may be the one or more lactic acid bacterial strain(s).

The fermented composition comprises high amount of lactic acid. Hence, the fermented composition may have a pH value in the range of pH 3-5, such as in the range of pH 3.5-4.5, e.g. in the range of pH 3.8-4.4, 3.85-4.1, 3.9-4.0, such as in the range of pH 4.0-4.3, e.g. about pH 4.2.

In an embodiment of the present invention the fermented composition may be mixed with one or more other fermented compositions comprising one or more other lactic acid bacterial strain(s) and/or one or more other plant materials. Such mixed fermented compositions may provide additional effects and/or synergistic effects.

The fermented composition according to the present invention may be used as an ingredient.

The fermented composition obtainable by the processes of the invention may form part of (or be) a food ingredient. In the present context, the terms "food" and "feed" may be used interchangeable, however, the term "food" refers specifically to eatable material suitable for human consumption, whereas the term "feed" refers specifically to eatable material suitable for animal consumption. The term "animal(s)" may include pigs, piglets, cattle, and horses, poultry such as chickens, turkeys, hens, geese, dogs, cats and ducks, and fish such as salmon and trout.

The food/feed ingredient may also form part of a food/feed product.

Preferably, the food/feed product comprises in the range 5-50% by weight of the dry feed/food ingredient, such as in the range 5-30%, such as 10-30% or such as 10-20%.

In a further aspect according to the present invention the fermented composition according to the present invention may be used in a food product; in a food supplement product; in an herbal medicine product; in a natural medicine product; in a medicinal product and/or as an adjuvant product accompanied by one or more other drugs. Preferably, the fermented composition according to the present invention may be used as an adjuvant product. In the present context the term "adjuvant" means contributing to or enhancing an existing medical regimen.

The inventors of the present invention surprisingly found that the composition according to the present invention has nutritional and/or pharmacological effects. Hence, a preferred embodiment of the present invention relates to a substance for use as a medicament comprising a fermented composition according to the present invention.

In the present context, the term "substance" relates to a drug, a complex material or a combination hereof that a may improve the well-being of a mammal. The complex material may preferably be the fermented composition according to the present invention.

In an embodiment of the present invention, the term "mammal" according to the present invention may be a human or an animal.

Preferably, the substance according to the present invention may be used in the treatment, alleviation, stabilisation or prophylaxis of autoinflammatory diseases in a mammal (such as a human or an animal), said substance comprises a fermented composition according to the present invention.

In an embodiment of the present invention, the autoinflammatory diseases may be selected from spondyloartropatia, spondylarthritis (in particular psoriasisartropetia or psoreasisartropatia); sclerosis (such as systemic sclerosis); allergy or intolerance (such as gluten allergy/intolerance and/or lactose allergy/intolerance); constipation; fatigue (such as cronic fatigue syndrome), inflammation, diarrhoea; inflammatory bowel diseases (such as crohn's disease, or cholitis ulcerosa in a mammal), skin diseases (such as pustulosis palmo et plantaris, Acne, Rosacia, and/or rinophyma), type 2 diabetes, and/or back pain with and without x-ray verified Modic changes (I and II).

Preferably, the autoinflammatory disease in a mammal may be selected from the group consisting of psoriasis, plaque psoriasis, guttate psoriasis, inverse psoriasis, pustular psoriasis, and erythrodermic psoriasis, sclerosis, such as systemic sclerosis; allergy or intolerance (such as gluten allergy/intolerance and/or lactose allergy/intolerance); inflammatory bowel diseases (such as crohn's disease, or cholitis ulcerosa) or type 2 diabetes.

The fermented product according to the present invention may be activating the intestinal micro flora or a mammal. In the present context the term "activating" relates to an increased activity of the intestinal micro flora, which exceeds the activity expected by the amount of lactic acid bacteria administered. In an embodiment of the present invention the intestinal micro flora may produce, after administering the fermented composition according the present invention, at least 2 times the amount of lactic acid relative to the normal amount of lactic acid produced, such as at least 3 times, e.g. at least 4 times, such as at least 5 times, e.g. at least 7.5 times, such as at least 10 times, e.g. at least 12.5 times, such as at least 15 times.

Researchers have found that often the cause of a disease, a condition, or an injury in a mammal is a result of an instable, abnormal and/or an infected intestinal flora, which prime the mammalian organism to react, e.g. in the form of a discomfort, a disease, a condition, or an injury. The inventors of the present invention surprisingly found that a fermented composition (or a substance) according to the present invention may be highly suitable for counter act or avoid such discomfort, disease, condition, or injury.

In the context of the present invention the term "treatment" relates to the use of an agent, such as the substance or the fermented composition according to the present invention, in an attempt to cure or mitigate a disease, a condition, or an injury in a mammal.

The term "alleviation" used in the present invention relates to the action of an agent, such as the substance or the fermented composition according to the present invention, to make a disease, a condition or an injury less intense and/or reduce symptoms in a mammal.

In the context of the present invention the term "prophylaxis" relates to the use of an agent, such as the substance or the fermented composition according to the present invention, in an attempt to prevent a disease, a condition or an injury in a mammal and/or for the protective treatment of a mammal.

The term "stabilising" used in the present invention relates to the use of an agent, such as the substance or the fermented composition according to the present invention, to stabilise the intestinal flora in a mammal for the improvement of the well-being (e.g. prevent, treat or alleviate a disease, a condition or an injury in a mammal). Many diseases, conditions and/or injuries in a mammal may be the result of an instable, abnormal and/or an infected intestinal flora. Thus, the term "stabilising" used in the present invention relates to the use of an agent, such as the substance or the fermented composition according to the present invention, to stabilise, normalise and fight infected intestinal flora in a mammal.

In an embodiment of the present invention the substance according to the present invention may be used in the treatment, alleviation, stabilisation or prophylaxis of autoinflammatory diseases such as spondyloartropatia, spondylarthritis (in particular psoriasisartropetia or psoreasisartropatia); sclerosis (such as systemic sclerosis); allergy or intolerance (such as gluten allergy/intolerance and/or lactose allergy/intolerance); constipation; fatigue (such as cronic fatigue syndrome), inflammation, diarrhoea; inflammatory bowel diseases (such as crohn's disease, or cholitis ulcerosa in a mammal), skin diseases (such as pustulosis palmo et plantaris, Acne, Rosacia, and/or rinophyma), type 2 diabetes, and/or back pain with and without x-ray verified Modic changes (I and II), wherein the substance comprises a fermented composition according to the present invention.

In an embodiment of the present invention, the mammal is a human or an animal, preferably, the animal is a domestic animal e.g. dog, cat, horse, cow, pig, chicken, sheep, or goat.

Psoriasis is a long-lasting autoimmune disease characterized by patches of abnormal skin. These skin patches are typically red, itchy, and scaly. They may vary in severity from small and localized to complete body coverage. The main types of psoriasis are plaque psoriasis, guttate psoriasis, inverse psoriasis, pustular psoriasis, and erythrodermic psoriasis. Furthermore, sequelae as a consequence of psoriasis are also encompassed by the definition of treatment, alleviation, stabilisation or prophylaxis of psoriasis. Sequelae that are associated with psoriasis are an increased risk of psoriatic arthritis, lymphomas, cardiovascular disease, Crohn's disease, Cholitis Ulserosa, chronic fatigue and depression.

In an embodiment of the present invention the fermented composition (or the substance) for the treatment, alleviation, stabilisation or prophylaxis of psoriasis comprising the combination of of Brassica spp. and Brown algae.

In an embodiment of the present invention wherein the Psoriasis Area and Severity Index (PASI) of a mammal suffering from psoriasis is reduced by 10% or more when the fermented composition or the substance according to the present invention is administered to a mammal, such as 20% or more, e.g. 30% or more, such as 40% or more, e.g. 50% or more, such as 60% or more, e.g. 70% or more, such as 80% or more, e.g. 90% or more, such as 95% or more, e.g. 100%.

The C-reactive protein (CRP) is a pentameric protein found in blood plasma of a mammal. The levels of C-reactive protein may rise in response to inflammation and is used as a marker of inflammation. Measuring and charting CRP values can prove useful in determining disease progress or the effectiveness of treatments.

Hence, one way to determine the extent or the severity of spondylarthritis, such as psoriasis, and in particular psoriatic arthritis, may be by measuring the level of C-reactive protein (CRP) in a mammal.

It is common that a maximum level (statistically evaluated) of CRP is accepted by the skilled person, whereby a mammal is still considered normal and healthy. In Denmark the health authorities have set a value of 8 (statistically evaluated) as the borderline for distinguishing between a healthy and a non-healthy person - based on the CRP value. In the present context the maximum level of CRP accepted for a normal/healthy mammal may be a CRP value below 8. Thus, a mammal having a CRP level below 8 are considered normal and healthy and mammals having a CRP value of 8 or above are considered abnormal/sick.

In the event said mammal is subjected to an inflammation the CRP level increases. Preferably, the CRP level of a mammal suffering from an inflammation may be at least 10 mg/l CRP, such as at least 15 mg/l CRP, e.g. at least 20 mg/l CRP, such as at least 25 mg/l CRP, e.g. at least 30 mg/l CRP, such as at least 40 mg/l CRP, e.g. at least 50 mg/l CRP, such as at least 75 mg/l CRP, e.g. at least 100 mg/l CRP.

In an embodiment of the present invention the CRP level in a mammal is lowered to a CRP level below 8 after administering a daily dose of the fermented composition according to the present invention, to the mammal. In particular, the CRP Level in a mammal is lowered to a CRP level below 8 in 5 months or less, such as in 4 months or less, e.g. in 3 months or less, such as in 2 months or less, e.g. in 1 months or less, such as in 25 days or less, e.g. in 22 days or less, such as in 20 days or less, e.g. in 18 days or less, such as in 17 days or less, e.g. in 16 days or less, such as in 15 days or less, e.g. in 14 days or less, such as in 12 days or less, e.g. in 10 days or less.

In a further embodiment of the present invention the CRP level in a mammal is lowered to a CRP level which is at most 75% of the CRP level before administering the fermented composition according the present invention, determined as an average value over a period of 10 days, such as at most 60%, e.g. at most 50%, such as at most 40%, e.g. at most 30%, such as at most 20%, e.g. at most 10%.

Sclerosis, in particular systemic sclerosis, is an autoimmune disease of the connective tissue. It is characterized by thickening of the skin caused by accumulation of collagen, and by injuries to small arteries. There are two forms of the disease. Limited cutaneous scleroderma affects only the face, hands, and feet. Diffuse cutaneous scleroderma covers more of the skin and may progress to visceral organs, including the kidneys, heart, lungs, and gastrointestinal tract.

Sequelae occurring as a consequence of Sclerosis are also encompassed by the definition of treatment, alleviation, stabilisation or prophylaxis of sclerosis.

In an embodiment of the present invention the fermented composition (or the substance) for the treatment, alleviation, stabilisation or prophylaxis of sclerosis, in particular systemic sclerosis, may preferably comprise Brassica spp., even more preferably, rape or rapeseed.

In a further embodiment of the present invention the fermented composition (or the substance) for the treatment, alleviation, stabilisation or prophylaxis of sclerosis, in particular systemic sclerosis, may preferably comprise a combination of Brassica spp. and brown algae.

In an embodiment of the present invention the scars in the skin of mammals suffering from sclerosis may become reduced by at least 20%, such as at least 30% e.g. at least 40%, such as at least 50% e.g. at least 60%, such as at least 70% e.g. at least 80%, such as at least 90% e.g. at least 95%.

Allergy, include a number of allergic diseases or conditions caused by hypersensitivity of the immune system to something in the environment. In some cases, the allergic reaction causes little but highly annoying problems in a mammals and in other situations the allergic reaction may be severe and immediate actions is needed. The allergic diseases may include hay fever, food allergies, atopic dermatitis, allergic asthma, allergic arthritis, atopic eczema, and anaphylaxis. Symptoms may include red eyes, an itchy rash, runny nose, shortness or trouble of breath, increased hart beating, suffocation, or swelling.

In an embodiment of the present invention the fermented composition (or the substance) for the treatment, alleviation, stabilisation or prophylaxis of allergy may preferably comprise a combination of Brassica spp. and brown algae.

In another embodiment of the present invention the fermented composition (or the substance) is suitable for the treatment, alleviation, stabilisation or prophylaxis of gluten allergy and/or lactose allergy.

Preferably, a mammal suffering from allergy, such as gluten allergy and/or lactose allergy, may, when taken the composition according to the present invention experience no allergic reaction eat normally afterwards.

Constipation refers to bowel movements that are infrequent or hard to pass. Constipation is a common cause of painful defecation. Severe constipation includes obstipation (failure to pass stools or gas) and fecal impaction, which can progress to bowel obstruction and become life-threatening. Constipation is common. In the general population rates of constipation varies from 2 to 30%. In old people living in care homes the rate of constipation is 50% to 75%. Furthermore, sequelae as a consequence of constipation are also encompassed by the definition of treatment, alleviation, stabilisation or prophylaxis of constipation. Sequelae, that are associated with constipation are spinal cord lesions, Parkinsons, colon cancer, anal fissures, proctitis, pelvic floor dysfunction, anismus, descending perineum syndrome, and Hirschsprung's disease

In an embodiment of the present invention the fermented composition (or the substance) for the treatment, alleviation, stabilisation or prophylaxis of constipation may preferably comprise Brassica spp., even more preferably, rape or rapeseed.

In an embodiment of the present invention the fermented composition (or the substance) is administered to a mammal in combination with one or more drugs in order to avoid the constipating side effect many medications have.

Preferably, constipation in a mammal may be treated or alleviated within 15 hours from intake of the fermented composition according to the present invention, such as within 10 hours, e.g. within 8 hours, such as within 6 hours, e.g. within 4 hours, such as within 3 hours, e.g. within 2 hours, such as within 1 hour.

Activation of intestinal gut flora, as described previously, relates to an increased activity of the intestinal micro flora, and may result in a more regular emptying of the intestine. By a more regular emptying of the intestine, the food has a shorter holding time in the intestine and the carbohydrates present in the food are easily converted by the intestinal micro flora, whereas the degradation of proteins from the food may be reduces and the tendency to create and store endotoxins in the intestines may be reduced.

Furthermore, as a consequence of activation of intestinal gut flora are also encompassed by the definition of treatment, alleviation, stabilisation or prophylaxis of activation of intestinal gut flora. Sequelae that are associated with activation of intestinal gut flora are e.g. obesity, and cancer.

The mammal may experience activation of the gut flora, by a more regular emptying of the intestine, within 15 hours from intake of the fermented composition according to the present invention, such as within 10 hours, e.g. within 8 hours, such as within 6 hours, e.g. within 4 hours, such as within 3 hours, e.g. within 2 hours, such as within 1 hour.

The fermented composition (or the substance) for the treatment, alleviation, stabilisation or prophylaxis of activation of the gut flora may preferably comprise a combination of Brassica spp. and brown algae.

Fatigue is a subjective feeling of tiredness which is distinct from weakness, and has a gradual onset. Unlike weakness, fatigue can be alleviated by periods of rest. Fatigue can have physical or mental causes. Physical fatigue is the transient inability of a muscle to maintain optimal physical performance, and mental fatigue is a transient decrease in maximal cognitive performance resulting from prolonged periods of cognitive activity. Furthermore, sequelae as a consequence of fatigue are also encompassed by the definition of treatment, alleviation, stabilisation or prophylaxis of fatigue. Sequelae that are associated with fatigue, in particular prolonged fatigue, are e.g. chronic fatigue, which may be a symptom of many diseases and conditions known to the skilled person.

In an embodiment of the present invention the mammal experiences a decreased fatigue, and/or increased energy, physical and/or mentally, by administering one dose daily of the fermented composition (or the substance) according to the present invention for 30 days or less, such as 25 days or less, e.g. 20 days or less, such as 15 days or less, e.g. 10 days or less, such as 5 days or less, e.g. 3 days or less, such as 2 days or less, e.g. 1 days or less.

In an embodiment of the present invention the fermented composition (or the substance) for the treatment, alleviation, stabilisation or prophylaxis of fatigue may preferably comprise a combination of Brassica spp. and brown algae.

Oligosaccharides, in particular found in breast milk stimulate the development of infants natural gut flora. However, some of these oligosaccharides are not synthesized by the mother but are provided to the mother via the diet or is synthesized by the mothers gut flora.

In an embodiment of the present invention the fermented product according to the present invention provides one or more oligosaccharides to the mother, which is then transferred to the infant. The transfer of the one or more oligosaccharides from the mother to the infant is done before birth or via breast milk after birth.

The fermented composition (or the substance) providing one or more oligosaccharides to the mother, which is then transferred to the infant may preferably comprise a combination of Brassica spp. and brown algae.

Inflammatory bowel disease (IBD) is a group of inflammatory conditions of the colon and small intestine. Diarrhoea, Crohn's disease and ulcerative colitis are the principal symptoms and types of inflammatory bowel disease. Not only does Crohn's disease affect the small intestine and large intestine, it can also affect the mouth, esophagus, stomach and the anus whereas ulcerative colitis primarily affects the colon and the rectum. Furthermore, sequelae as a consequence of inflammatory bowel disease are also encompassed by the definition of treatment, alleviation, stabilisation or prophylaxis of inflammatory bowel disease. Sequelae that are associated with inflammatory bowel disease are arthritis, pyoderma gangrenosum, primary sclerosing cholangitis, non-thyroidal illness syndrome (NTIS), and fatigue.

Furthermore, diarrhoea may arise in patients subjected to a gastric bypass operation. Gastric bypass operation refers to a surgical procedure in which the size of the stomach is reduced by being divided into a small upper pouch and a much larger lower "remnant" pouch and then the small intestine is rearranged to connect to both. The operation may normally be prescribed to treat morbid obesity (defined as a body mass index greater than 40), type 2 diabetes, hypertension, sleep apnoea, and other comorbid conditions.

When the gastric bypass patient eats a sugary food, the sugar passes rapidly into the intestine, where it gives rise to a physiological reaction called dumping syndrome. The body will flood the intestines in an attempt to dilute the sugars resulting in diarrhoea. Hence, patients subjected to gastric bypass operations often suffer from diarrhoea and thereby risk a malnutritional stage.

In an embodiment of the present invention treatment of diarrhoea (either arising from Inflammatory bowel disease (IBD), e.g. Crohn's disease, gastric bypass operations, or from infection by bacteria, such as *E. coli,* virus or parasites) in a mammal may be obtained by administering one dose daily of the fermented composition (or the substance) according to the present invention within 5 days or less, such as 4 days or less, e.g. 3 days or less, such as 2 days or less, e.g. 1 days or less, such as 20 hours or less, e.g. 15 hours or less, such as 10 hours or less, e.g. 5 hours or less, such as within 2 hours or less.

In the present contest the term "treatment of diarrhoea in a mammal" relates to the subjective opinion of the mammal of the improved health and/or the objective inspection of the stool obtained from the mammal, e.g. using the Bristol Stool Chart.

In an embodiment of the present invention alleviation of diarrhoea in a mammal may be obtained by administering one dose daily of the fermented composition (or the substance) according to the present invention within 5 days or less, such as 4 days or less, e.g. 3 days or less, such as 2 days or less, e.g. 1 days or less, such as 20 hours or less, e.g. 15 hours or less, such as 10 hours or less, e.g. 5 hours or less, such as within 2 or less.

In the present contest the term "alleviation of diarrhoea in a mammal" relates to the subjective opinion of the mammal of the improved health and/or the objective inspection of the stool obtained from the mammal, e.g. using the Bristol Stool Chart.

In an embodiment of the present invention the fermented composition (or the substance) for the treatment, alleviation, stabilisation or prophylaxis of inflammatory bowel disease may preferably comprise a combination of Brassica spp. and brown algae.

The fermented composition (or substance) according to the present invention, have surprisingly shown to be effective against bacterial infection.

In an embodiment of the present invention the fermented composition (or the substance) may be used for the treatment, alleviation, stabilisation or prophylaxis of bacterial infection may preferably comprise seaweed/algae; or a combination of Brassica spp. and seaweed/algae.

In the present context, the term "bacterial infection" relates to infections caused by either gram positive bacteria or gram negative bacteria. Preferably, the bacterial infection referred to in the present invention may be caused by gram positive bacteria, in particular *Staphylococcus,* preferably Staphylococcus aureus, and/or *Clostridium,* preferably *Clostridium perfringens* and Clostridium difficile.

As mentioned, one such bacterial infection may be a *staphylococcus* infection, in particular a *Staphylococcus aureus* infection, which may result in e.g. furunculosis, or even worse in chronic furunculosis.

Furunculosis is most common in animals, such as dogs or cats, but it may also occur in humans. Animals and humans suffering from furunculosis may develop furuncle, which is a deep folliculitis (an infection and inflammation of one or more hair follicles).

Furuncles are bumpy, red, pus-filled lumps around a hair follicle that are tender, warm, and very painful. They range from pea-sized to golf ball-sized. A yellow or white point at the center of the lump can be seen when the boil is ready to drain or discharge pus. In a severe infection, an individual may experience fever, swollen lymph nodes, and fatigue.

Furuncles may appear on the buttocks or near the anus, the back, the neck, the stomach, the chest, the arms, legs or feet, or even in the ear canal.

*Staphylococcus aureus* may spread to different parts of the body via the bloodstream (bacteremia), causing bacterial infections (or sequelaes) like wound infections, abscesses, osteomyelitis, endocarditis, or pneumonia, that may severely harm or even kill the infected animal or human.

*Staphylococcus aureus* strains may also produce enzymes and exotoxins that likely cause or increase the severity of certain diseases. Such diseases are also considered sequelae as a consequence of bacterial infection and are also encompassed by the definition of treatment, alleviation, stabilisation or prophylaxis of bacterial infection, and include food poisoning, septic shock, toxic shock syndrome, and scalded skin syndrome. Almost any organ system can be infected by *Staphylococcus aureus.*

Another bacterial infection may be a *Clostridium* infection, which may result in e.g. food poisoning, sepsis or myonecrosis, such as gas gangrene. The *Clostridium* infection may in particular be caused by *Clostridium perfringens* or Clostridium difficile.

Myonecrosis, e.g. gas gangrene, is a condition of necrotic damage, specific to muscle tissue. It is often seen in infections with *Clostridium perfringens* that cause myonecrosis by producing alpha toxins. This alpha toxin is a lethal toxin and also known as phospholipase C (lecithinase). It increases vascular permeability and produces necrotizing activity. The toxins secreted are very powerful and destroy nearby tissue and generating gas at the same time.

A *Clostridium* infection may be treated, alleviated, stabilised by administering a fermented composition (or the substance) according to the present invention to the infected mammal. The fermented composition (or the substance) according to the present invention may preferably comprise a combination of Brassica spp. and brown algae.

In an embodiment of the present invention the fermented composition (or the substance) is administered to a mammal (an animal or a human) in combination with one or more drugs in order to avoid the bacterial infection; the bacterial infection side effects; or the side effects many medications may have.

Preferably, bacterial infections in a mammal, such as bacterial infection caused by *Staphylococcus aureus and/or* by *Clostridium perfringens* or *Clostridium difficile,* may be treated or alleviated within 40 days, such as within 30 days, e.g. within 20 days, such as within 15 days, e.g. within 12 days, such as within 10 days.

The fermented composition or the substance may be use as a blood cholesterol reducing agent, a triglyceride reducing agent, reducing liver damages, and/or hypertension reducing agent, wherein the substance comprises a fermented composition according to the present invention.

Cholesterol is a complex group of compound because some are considered detrimental to the mammal body and for years, some cholesterol compounds have been responsible for various diseases and resulted in deaths of humans, whereas other cholesterol compounds are essential for animal life.

The blood cholesterol may be reduced, by administering one dose daily of the fermented composition (or the substance) according to the present invention for 200 days or less, such as 150 days or less, e.g. 100 days or less, such as 75 days or less, e.g. 50 days or less, such as 40 days or less, e.g. 30 days or less, such as 20 days or less, e.g. 10 days or less.

The fermented composition (or the substance) used for reducing the blood cholesterol level in a mammal may preferably comprise *Brassica spp.,* such as rape species; seaweed/algae; or a combination of *Brassica spp.,* such as rape species, and seaweed/algae.

The total cholesterol level is the overall amount of cholesterol found in the blood. The total cholesterol consists of low-density lipoproteins (LDL), also called "bad" cholesterol, which is the cholesterol that blocks blood vessels and increases the risk of heart disease. It also consists of high-density lipoproteins (HDL), the "good" cholesterol that helps protect from heart diseases. The higher the HDL, the better. Total cholesterol also includes a triglycerides count. These triglycerides are another type of fat that can build up in the body. High levels of triglycerides and low levels of HDL raise the risk for heart diseases.

The fermented composition (or the substance) according to the present invention may reduce the concentration of total cholesterol in the blood of a mammal. Preferably, the total cholesterol may be reduced, relative to the concentration of total cholesterol in mammals not taken cholesterol reducing medicine with at least 2%, such as at least 5%, e.g. at least 10%, such as at least 15%, e.g. at least 20%, such as at least 25%, e.g. at least 30%.

A reduction in total cholesterol may be obtained, by administering one dose daily of the fermented composition (or the substance) according to the present invention, for 200 days or less, such as 150 days or less, e.g. 100 days or less, such as 75 days or less, e.g. 50 days or less, such as 40 days or less, e.g. 30 days or less, such as 20 days or less, e.g. 10 days or less.

The fermented composition (or the substance) used for reducing the total cholesterol level in a mammal may preferably comprise *Brassica spp.,* such as rape species; seaweed/algae; or a combination of *Brassica spp.,* such as rape species, and seaweed/algae.

LDL cholesterol are considered being "bad" cholesterol since LDL particles pose a health risk for cardiovascular disease when they invade the endothelium and become oxidized. Increasing concentrations of LDL particles are strongly associated with increasing rates of accumulation of atherosclerosis within the walls of arteries over time. Increased concentrations of LDL are known to increase the incidence and the risk of e.g. cardiovascular disease, stroke, and other vascular disease complications.

The fermented composition (or the substance) according to the present invention may reduce the concentration of LDL cholesterol in the blood of a mammal. Preferably, the LDL cholesterol may be reduced, relative to the concentration of LDL cholesterol in mammals not taken cholesterol reducing medicine with at least 10%, such as at least 15%, e.g. at least 20%, such as at least 25%, e.g. at least 30%, such as at least 35%, e.g. at least 40%, such as at least 45%, e.g. at least 50%, such as at least 60%, e.g. at least 70%.

The reduction in LDL cholesterol may be obtained, by administering one dose daily of the fermented composition (or the substance) according to the present invention, for 200 days or less, such as 150 days or less, e.g. 100 days or less, such as 75 days or less, e.g. 50 days or less, such as 40 days or less, e.g. 30 days or less, such as 20 days or less, e.g. 10 days or less.

The fermented composition (or the substance) used for reducing the LDL cholesterol level in a mammal may preferably comprise *Brassica spp.,* such as rape species; seaweed/algae; or a combination of *Brassica spp.,* such as rape species, and seaweed/algae.

HDL cholesterol is referred to as "the good" cholesterol or "healthy" cholesterol because they can transport fat molecules out of artery walls, reduce macrophage accumulation, and thus help prevent or even regress atherosclerosis and decrease the incidence and the risk of e.g. cardiovascular disease, stroke, and other vascular disease complications.

The fermented composition (or the substance) according to the present invention may remain the level or even increase the concentration of HDL cholesterol in the blood of a mammal. Preferably, the HDL cholesterol may be increased, relative to the concentration of LDL cholesterol in mammals not taken cholesterol reducing medicine with at least 2%, such as at least 5%, e.g. at least 8%, such as at least 10%, e.g. at least 15%, such as at least 20%, e.g. at least 25%, such as at least 30%.

The HDL cholesterol may be maintained or increased, by administering one dose daily of the fermented composition (or the substance) according to the present invention for 200 days or less, such as 150 days or less, e.g. 100 days or less, such as 75 days or less, e.g. 50 days or less, such as 40 days or less, e.g. 30 days or less, such as 20 days or less, e.g. 10 days or less.

The fermented composition (or the substance) used for maintaining or increasing the HDL cholesterol level in a mammal may preferably comprise *Brassica spp.,* such as rape species; seaweed/algae; or a combination of *Brassica spp.,* such as rape species, and seaweed/algae.

Triglycerides are the form in which most fat exists in food and in the body. In the human body, high levels of triglycerides in the bloodstream have been linked to atherosclerosis and, by extension, the risk of heart disease and stroke.

The fermented composition (or the substance) according to the present invention may reduce the concentration of triglycerides in the blood of a mammal. Preferably, the triglycerides may be reduced, relative to the concentration of triglycerides in mammals not taken the composition (or substance) according to the present invention, with at least 10%, such as at least 15%, e.g. at least 20%, such as at least 25%, e.g. at least 30%, such as at least 35%, e.g. at least 40%, such as at least 45%, e.g. at least 50%, such as at least 60%, e.g. at least 70%.

The reduction in blood triglycerides may be obtained, by administering one dose daily of the fermented composition (or the substance) according to the present invention, for 200 days or less, such as 150 days or less, e.g. 100 days or less, such as 75 days or less, e.g. 50 days or less, such as 40 days or less, e.g. 30 days or less, such as 20 days or less, e.g. 10 days or less.

The fermented composition (or the substance) used for reducing the triglyceride level in a mammal may preferably comprise *Brassica spp.,* such as rape species; seaweed/algae; or a combination of *Brassica spp.,* such as rape species, and seaweed/algae.

AST (aspartate aminotransferase) and ALT (alanine aminotransferase) and ALP (Alkaline phosphatase) are traditionally used as biomarkers for liver damages. Increased levels of AST, ALT and/or ALP is indicative of liver damages. Liver function tests are used to give information about the state of a patient's liver. Most liver damages or liver diseases cause only mild symptoms initially, but these diseases must be detected early.

The inventors of the present invention surprisingly found that AST (aspartate aminotransferase) and ALT (alanine aminotransferase) and ALP (Alkaline phosphatase) was significantly reduced in patients taken the fermented composition (or the substance a) according to the present invention.

The fermented composition (or the substance) according to the present invention may reduce the concentration of AST and/or ALT and/or ALP in the blood of a mammal. Preferably, the total cholesterol may be reduced, relative to the concentration of total cholesterol in mammals not taken cholesterol reducing medicine with at least 2%, such as at least 5%, e.g. at least 10%, such as at least 15%, e.g. at least 20%, such as at least 25%, e.g. at least 30%.

A reduction in AST and/or ALT and/or ALP may be obtained, by administering one dose daily of the fermented composition (or the substance) according to the present invention, for 200 days or less, such as 150 days or less, e.g. 100 days or less, such as 75 days or less, e.g. 50 days or less, such as 40 days or less, e.g. 30 days or less, such as 20 days or less, e.g. 10 days or less.

The fermented composition (or the substance) used for reducing the AST and/or ALT and/or ALP in the blood of a mammal may preferably comprise *Brassica spp.,* such as rape species; seaweed/algae; or a combination of *Brassica spp.,* such as rape species, and seaweed/algae.

One sequela of damaged liver is hypertension, also known as high blood pressure (HBP). Due to the improved effect on the liver as mentioned a reduced blood pressure may be provided in patients taken the fermented composition (or the substance) according to the present invention. Some of these effects may be a long term medical condition in which the blood pressure in the arteries is persistently elevated. Long term high blood pressure, is a major risk factor for coronary artery disease, stroke, heart failure, peripheral vascular disease, vision loss, and chronic kidney disease.

One dose or a single dose may relate to 50 gram fermented product according to the present invention or less, such as 40 g or less, e.g. 30 gram or less, such as 25 gram or less, e.g. 20 gram or less, e.g. 15 gram or less, such as 10 gram or less.

The substance and/or the fermented composition may be used as an adjuvant product accompanied by one or more other drugs.

Without being bound by any theory, the fermented composition and/or the substance may comprise a dual function within the combined product according to the present invention. The dual function may lay in a first aspect of treating, stabilising and/or alleviating and/or in a second aspect of the prophylaxis of a diseases, conditions and/or injuries in a mammal.

It should be noted that embodiments and features described in the context of one of the aspects of the present invention also apply to the other aspects of the invention.

The invention will now be described in further details in the following non-limiting examples.

**Examples**

Example 1

Preparation of a fermented composition comprising rapeseed according to the present invention.

The present invention was exemplified by mixing about 80% rapeseed meal (having an average diameter of about 2 mm), about 14% wheat 1% potato flakes (vegetable source), and 5% Perlac (carbohydrate source) in a fermentation tank. The moisture content was adjusted to about 40% humidity and a combination of the lactic acid-producing bacteria Pediococcus pentosaceus, Pediococcus acidilactici and Lactobacillus plantarum was added. The air intake to the fermentation tank was taken directly from the air in the room wherein the fermentation tank was situated. The ingredients were mixed at room temperature and the temperature was slowly increased from the heat generated from the fermentation to a steady state temperature of about 42°C.

After 20 hours of fermentation the fermentation broth had a pH of 4.2 and a lactic acid concentration of about 140 mM. The fermented composition had a bacterial count of $3.6 \times 10^6$ CFU/gram and analyzing the fermented composition showed very small amounts of acetic acid and ethanol were produced, hence the mixture was considered being 95-100% homofermentative. The glucosinolate content after fermentation was 16.5 μmol/g, whereby about 50% of the glycosinolate naturally present in the rapeseed meal was retained.

The resulting fermented composition was subjected to spin flash drying in order to protect the lactic acid bacteria and keep a high number of viable cells.

Example 2

A method according to the present invention for providing a fermented composition comprising a combination of 20% seaweed (*Laminaria saccharina*) and 80% rapeseed meal.

Seaweed (*Laminaria saccharina*), was pre-treated to an average diameter of about 1 mm, and mixed with a combination of the lactic acid-producing bacteria Pediococcus pentosaceus, Pediococcus acidilactici and Lactobacillus plantarum in a fermentation tank and the moisture content was adjusted to about 40% humidity. The air intake to the fermentation tank was taken directly from the air in the room wherein the fermentation tank was situated. The fermentation was performed at a temperature of about 30°C.

After 3 days of fermentation of the seaweed, rapeseed meal, which was pre-treated to about 2 mm, was added to the fermented composition comprising seaweed, in a ratio of rapeseed meal and seaweed is 80:20 (on a weight basis), together with a combination of the lactic acid-producing bacteria Pediococcus pentosaceus, Pediococcus acidilactici and Lactobacillus plantarum in a fermentation tank and the fermentation was continued for 3 days at 25°C. The resulting fermentation broth had a bacterial count of $3.0 \times 10^6$ CFU/gram, a pH of 4.2 and a lactic acid concentration of about 140 mM.

The resulting fermented composition comprising the combination of seaweed, rapeseed meal and lactic acid bacteria, was subjected to spin flash drying in order to protect the lactic acid bacteria and keep a high number of viable cells.

Example 3

2 patients suffering from gluten allergy and 1 patient suffering from lactose intolerance was taken about 25 g of the fermented composition obtained in example 1 per day. After taken the fermented composition none of the patients experienced any discomforts or allergic reactions and all three patients could eat without restrictions. One patient took a stopped taken the fermented composition and the allergic reaction came back. When starting taken the fermented composition again the patient experienced no discomforts or allergic reactions and could eat without restrictions again.

Example 4

8 patients suffering from constipation was taken a single dose of about 25 g of the fermented composition obtained in example 1. After 1-3 hours all patients experienced relieve in constipation and improved faecal flow.

Example 5

1 patient suffering from diarrhoea arising from a bacterial infection by *E. Coli*, was taken a single dose of 20 g of the fermented composition obtained in example 1. After 2 hours the patient got firmer stool and started feeling better. After 10 hours the stool became looser again the symptoms of diarrhoea came back. The patient took another 20 g of the fermented composition obtained in example 1 and the patient again got firmer stool and felt better.

Example 6

2 patients suffering from diarrhoea arising from the Inflammatory bowel disease (IBD), Crohn's disease, and 2 patients suffering from from the Inflammatory bowel disease (IBD), colitis ulcerosa, was, not responding to traditional medical treatment, were taken a single dose of 15 g of the fermented composition obtained in example 2. After 2 hours the patients got firmer stool and started feeling better with significantly less stomach pain.

Example 7

1 patient 58 years old suffering from HLA B27 associated spondylarthritis, psoriatic arthritis, was for about 4½ years receiving a normally prescribed treatment comprising DMARD treatment (treatment with Disease modifying anti Rheumatoc Drugs) such as treatment with an anti-TNF alpha drug and methotrexate.

A single daily dose of 5-10 gram of the dry fermented composition obtained in Example 2 was administered to the patient, together with full dose of the normally prescribed medicine, the DMARD treatment together with a TNF alpha antibody and methotrexate.

During the period of 4½ years of treatment with the normally prescribed medicine the CRP levels was fluctuating between about 20 mg/l to about 110 mg/l. However, after 17 days of supplementing the normally prescribed medicine with the fermented composition the CRP levels of the patient was lowered to about 3 mg/l and normalised (see figure 1).

Furthermore, the Psoriasis Area and Severity Index (PASI) were reduced by about 70%.

Example 8

Laboratory experiments were made comparing different compositions effect against the Gram positive bacteria *Clostridium perfringens.* Three compositions were tested:

1) A composition according to the present invention, wherein the plant material is seaweed, grinded and suspended in water at a concentration of 0.25 g/ml. After vigorously stirring the solids and the supernatant is used for the test,

2) A composition according to the present invention, wherein the plant material is rape seed, grinded and suspended in water at a concentration of 0.25 g/ml. After vigorously stirring the solids and the supernatant is used for the test,

3) A control comprising water.

All samples were provided with approximately $10^6$ CFU/ml of *Clostridium perfringens* and a McFarland turbidity of about 0.5. The samples are incubated for about 24 hours at 37°C.
From the visual inspection it was clear that the composition comprising seaweed (1) inhibited growth of *Clostridium perfringens,* whereas, growth of *Clostridium perfringens* was found in both the sample comprising rape seed (2) and the control sample comprising water (3).

Example 9

1 patient suffering from severe acne was taken a single dose of 20 g of the fermented composition obtained in example 1. After a period of 14 days, the skin manifestations were completely gone.
The same patient also suffered form cronic back pain. However, after one month of the acne treatment with the fermented composition of example 1, the back pain surprisingly disappeared completely. After seponation of the fermented composition in one week the back pain returned. After restarting administering a single daily dose of 20 g of the fermented product of example 1 the back pain was gone again.

Example 10

In order to identify potential differences and products in the plant extracts they were subjected to untargeted metabolomics analysis by Ultra High Pressure Liquid Chromatography coupled to a Q-ToF mass spectrometer (UHPLC-Q-ToF-MS).
Chromatography was performed on a Dionex UltiMate® 3000 Quaternary Rapid Separation UHPLC+ focused system (Thermo Fisher Scientific, Germering, Germany).
Separation was achieved on a Kinetex 1.7u XB-C18 column (100 x 2.1 mm, 1.7 $\mu$m, 100 Å, Phenomenex, Torrance, CA, USA). Formic acid (0.05%) in water and acetonitrile (supplied with 0.05% formic acid) were employed as mobile phases A and B, respectively.
Gradient conditions were as follows: 0.0-0.5 min, 2% B; 0.5-14.0 min 2-20% B; 14.0-20.0 min 20-45% B, 20.0-24.5 min 45-100% B, 24.5-26.5 min 100%, 26.5-26.55 min 100-2% B and 26.55-30.0 min 2% B. The mobile phase flow rate was 300 $\mu$l min-1. The column temperature was maintained at 25°C. Four wavelengths (205 nm, 220 nm, 250 nm and 390 nm) were monitored by a UV-VIS detector.
The liquid chromatography was coupled to a Compact micrOTOF-Q mass spectrometer (Bruker, Bremen, Germany) equipped with an electrospray ion source (ESI) operated in positive or negative ionization mode. The ion spray voltage was maintained at -3900 V in negative mode. Dry temperature was set to 250°C and dry gas flow was set to 8 L min-1. Nebulizing gas was set to 2.5 bar and collision energy to 15 eV. Nitrogen was used as dry gas, nebulizing gas and collision gas.
The m/z range was set to 50-1400. AutoMSMS mode was used to obtain MS and MS/MS spectra of the three most abundant ions present at each time point with smart exclusion to also include less abundant ions. All files were calibrated based on compound spectra collected from Na+-formiate clusters at the beginning of each run.
Methanol extract samples (80% methanol) of rape species (rape seed) according to the present invention, seaweed (*Laminaria spp.*) according to the present invention, the combination of rape species (rape seed) and seaweed (*Laminaria spp.*) according to the present invention; non-fermented rape species (rape seed); and non-fermented seaweed (*Laminaria spp.*) were run in autoMSMS mode to acquire MS and MS/MS spectra of molecules present in the different extracts. The m/z range was set to 50-1400. Na+-formiate clusters were used as calibrant and injected at the beginning of each sample run. All files were automatically calibrated based on the compound spectra collected from the Na$^+$-formiate clusters by post processing.

Results

Analyzing the fermented combination product (rape and seaweed) showed a dominating peak has a "mass to charge ratio" (m/z) of (in negative mode):

$$MS1\ mass = 311.2228$$

MS2 masses = 223.1694; 87.0436; 235.1666; 224.1725; 275.2005
Retention time: 22.52 min (under the set conditions)
It is estimated that this compound is a fatty acid, in particular a modified fatty acid.

A second dominating peak was found having a "mass to charge ratio" (m/z) of (in negative mode):
MS1 mass =225.0772

Retention time: 11.86 min (under the set conditions)
From comparative studies with pure samples of HDMPPA this second dominating peak showed to be HDMPPA.

Analyzing the fermented rape showed a dominating peak has a "mass to charge ratio" (m/z) of (in negative mode):
MS1 mass =225.0772

Retention time 11.86 min (under the set conditions)
From comparative studies with pure samples of HDMPPA this second dominating peak showed to be HDMPPA.

Analysing the fermented seaweed (Laminaria spp.); the fermented rape (rape seed); and the unfermented seaweed (Laminaria spp.) and un-fermented rape (rape seed) showed no significant peakes similar to the above results (see figure 2 and 3).

As it appears from the chromatogram in figure 2, a significant amount of HDMPPA (3-(4'-hydroxyl-3',5'-dimethoxyphenyl)propionic acid) in fermented rapeseed/seaweed (1) and fermented rapeseed (2) may be obtained relative to non-fermented rapeseed (3). From this chromatogram it is shown that fermented rapeseed/seaweed (1) has significant more HDMPPA relative to fermented rapeseed (2), which has significant more HDMPPA relative to non-fermented rapeseed (2).

As it appears from the chromatogram in figure 3, a compound may be found in significant amounts in the fermented rapeseed/seaweed (1); whereas fermented rapeseed (2); non-fermented seaweed (3); and non-fermented rapeseed (4), has no or insignificant amounts of the compound. In combination with further analysis of molecular masses MS1 and MS2 of the fermented rapeseed/seaweed (1) it is suggested the compound found in the fermented rapeseed/-seaweed (1); compared to the fermented rapeseed (2); non-fermented seaweed (3); and non-fermented rapeseed (4) was a fatty acid molecule, such as a modified fatty acid molecule.

Example 11

The effect on cholesterol and liver damages of the fermented composition according to the present invention were tested on two different breeds of sows, 1) the polish large white and 2) the polish landrace. The tests were made on both pregnant and lactating sows.

The following groups of sows and feed were made:

K1 Pregnant: polish large white sows fed normal feed without the fermented composition.
K2 Pregnant: polish landrace sows fed normal feed without the fermented composition.
K1 Lactating: polish large white sows fed normal feed without the fermented composition.
K2 Lactating: polish landrace sows fed normal feed without the fermented composition.
D1 Pregnant: polish large white sows fed mixed feed comprising 4% of the fermented composition as obtained in example 1.
D2 Pregnant: polish landrace sows fed mixed feed comprising 4% of the fermented composition as obtained in example 1.

D1 Lactating: polish large white sows fed mixed feed comprising 4% of the fermented composition as obtained in example 1.
D2 Lactating: polish landrace sows fed mixed feed comprising 4% of the fermented composition as obtained in example 1.

After 150 days of feeding blood samples were obtained and analysed for:

Concentration of total cholesterol
Concentration of LDL cholesterol
Concentration of HDL cholesterol
Concentration of triglycerides
Concentration of AST
Concentration of ALT
Concentration of ALP

Results from cholesterol and fat

The results from determining total cholesterol, LDL cholesterol, HDL cholesterol and triglycerides are shown in figure 4.

It is surprisingly shown (see figure 4, solid bars and arrows) that the total cholesterol is significantly reduced in sows regardless of the breed and whether the sow is lactating or pregnant. Furthermore, it seems like the reduction is more pronounced in polish large white than in polish landrace and lactating sows than in pregnant sows. Pregnant polish large white sows feed with the composition obtained in example 1 experienced a total cholesterol reduction (A) of 15%, whereas Pregnant polish landrace sows feed with the composition obtained in example 1 only experienced a total cholesterol reduction (B) of 10%. However, the most significant reduction was obtained for lactating polish large white sows feed with the composition obtained in example 1, which experienced a total cholesterol reduction (C) of 25%.

In respect of LDL cholesterol, the inventor surprisingly found that the concentration of LDL cholesterol (the "bad" cholesterol) is significantly reduced in sows regardless of the breed and whether the sow is lactating or pregnant (see figure 4, horizontally hatched bars and arrows). Furthermore, it seems like the reduction is more pronounced in polish large white than in polish landrace and lactating sows than in pregnant sows. Pregnant polish large white sows feed with the composition obtained in example 1 experienced a LDL cholesterol reduction (G) of 58%, whereas Pregnant polish landrace sows feed with the composition obtained in example 1 only experienced a LDL cholesterol reduction (H) of 36%. However, the most significant reduction was obtained for lactating polish large white sows feed with the composition obtained in example 1, which experienced a LDL cholesterol reduction (J) of 72%, and lactating polish landrace sows feed with the composition obtained in example 1 experienced a LDL cholesterol reduction (K) of 49%.

In respect of triglycerides, the inventor found that the concentration of triglycerides is also significantly reduced in sows regardless of the breed and whether the sow is lactating or pregnant (see figure 4, dashed bars and arrows). Pregnant polish landrace sows feed with the composition obtained in example 1 only experienced a triglycerides reduction (D) of 21%; lactating polish large white sows feed with the composition obtained in example 1, experienced a triglycerides reduction (E) of 28%, and lactating polish landrace sows feed with the composition obtained in example 1 experienced a triglycerides reduction (F) of 38%.

A further surprising finding made by the present inventor is that sows fed a mixed feed comprising the composition as obtained in example 1, showed to maintain or even increase the concentration of HDL cholesterol (the "good" cholesterol) in the blood of sows.

The test showed (see figure 4, vertically hatched bars and arrows) that pregnant polish large white sows feed with the composition obtained in example 1 experienced a HDL cholesterol increase of 3%; pregnant polish landrace sows feed with the composition obtained in example 1 experienced a HDL cholesterol increase of 10%; lactating polish large white sows feed with the composition obtained in example 1, experienced a HDL cholesterol increase of 10%; and lactating polish landrace sows feed with the composition obtained in example 1 experienced a HDL cholesterol increase of 35%.

Hence, the fermented composition (or the substance) according to the present invention is highly active in lowering the concentration of fat and "bad" cholesterol particles whereas the concentration of "good" cholesterol particles are increased and other side effect from cholesterol lowering agents are avoided. Such side effects may include intestinal problems, liver damage, muscle inflammation, high blood sugar and type 2 diabetes.

Results regarding liver damage analysis

The results from determining the concentrations of AST, ALT, and ALP are listed in the below table 1.

Table 1. AST, ALT and ALP measurements from feeding different breeds of lactating and pregnant sows with or without the fermented composition as provided in example 1.

| Indicator | K1 | D1 | P-Value | K2 | D2 | P-value | KxD |
|---|---|---|---|---|---|---|---|
| PREGNANCY SOWS | | | | | | | |
| AST (U/L) | 32.45 | 24.80 | 0.032 | 44.31 | 38.85 | 0.039 | 0.018 |
| ALT (U/L) | 41.31 | 33.45 | 0.041 | 62.58 | 60.61 | 0.410 | 0.043 |
| ALP (U/L) | 175.67 | 169.99 | 0.231 | 125.43 | 130.52 | 0.309 | 0.032 |
| LACTATION SOWS | | | | | | | |
| AST (U/L) | 26.44 | 16.17 | 0.023 | 44.33 | 27.60 | 0.028 | 0.025 |
| ALT (U/L) | 29.42 | 24.89 | 0.058 | 23.74 | 20.55 | 0.122 | 0.042 |
| ALP (U/L) | 152.45 | 119.29 | 0.032 | 130.42 | 91.94 | 0.036 | 0.036 |

As shown in table 1 sows fed with a mixed feed comprising 4% fermented composition as obtained in example 1 significantly reduces the concentration of the biomarkers AST and ALT. ALP levels in blood may rise with large bile duct obstruction, intrahepatic cholestasis, or infiltrative diseases of the liver, however, when feeding the composition as obtained in example 1, the ALP is also significantly reduced.

Hence, the fermented composition (or the substance) according to the present invention is highly active in lowering the concentration AST, ALT and ALP and other side effect from traditional medication may be avoided.

**References**

WO 2013/029632
WO 2014/131422
WO 2014/206419
Jin-Woo Jeong et al., Journal of Medicinal Food, 18 (6) 2015, 677-684
Hyun Ju Kim et al., J. Agric. Food Chem., 2007, 55, 10486-10492
Jeong sook Noh et al., British Journal of Nutrition; (2013), 109, 17-24
Ye-Rang Yun et al., Journal of Medicinal Food, 17 (8) 2014, 886-893

**Claims**

1.  A substance for use in the treatment, alleviation, stabilising or prophylaxis of an autoinflammatory disease in a mammal (such as a human or an animal), said substance comprises a fermented composition comprising a combination of Brassica spp. and seaweed or algae; and one or more lactic acid bacterial strain(s), wherein the seaweed or algae is brown algae and wherein the fermented composition is provided by an essentially homofermentative process.

2.  A substance for use according to claim 1, wherein the autoinflammatory disease in a mammal is selected from the group consisting of spondyloartropatia, spondylarthritis (in particular psoriasisartropetia or psoreasisartropatia); sclerosis (such as systemic sclerosis); allergy or intolerance (such as gluten allergy/intolerance and/or lactose allergy/intolerance); constipation; fatigue (such as cronic fatigue syndrome), inflammation, diarrhoea; inflammatory bowel diseases (such as crohn's disease, or cholitis ulcerosa in a mammal), skin diseases (such as pustulosis palmo et plantaris, Acne, Rosacia, and/or rinophyma), and/or back pain with and without x-ray verified Modic changes (I and II).

3.  A substance for use according to anyone of claims 1 or 2, wherein the autoinflammatory disease in a mammal is selected from the group consisting of psoriasis, plaque psoriasis, guttate psoriasis, inverse psoriasis, pustular psoriasis, and erythrodermic psoriasis, sclerosis, such as systemic sclerosis; allergy or intolerance (such as gluten allergy/intolerance and/or lactose allergy/intolerance) or inflammatory bowel diseases (such as crohn's disease, or cholitis ulcerosa).

4. The substance for use according to anyone of claims 1-3, wherein the substance is an adjuvant product accompanied by one or more other drugs.

5. The substance for use according to anyone of claims 1-4, wherein the fermented composition has a pH below pH 6.5, such as below pH 6.0, e.g. below pH 5.5, such as below pH 5.0, e.g. in the range of pH 3.0-6.5, such as in the range of pH 3.0-6.0, e.g. in the range of pH 3.1-5.5, such as in the range of pH 5.0-3.2, such as in the range 3.3- 4-2, such as 3.4-4.0, such as 3.5-3.8, such as 3.7-4.2, such as 3.7-4.0, or such as 3.8-4.2.

6. The substance for use according to anyone of claims 1-5, wherein the fermented composition has a lactic acid concentration of at least 50 mM, such as at least 100 mM, such as 100-1000 mM, such as 100-500 mM, such as 100-300 mM, such as 100-200 mM, such as 150-500 mM, such as 200-500 mM or such as 300-500, mM lactic acid.

7. The substance for use according to anyone of claims 1-6, wherein the brown algae is selected from kelps, *Laminaria saccharina, Laminaria digitata,* and/or *Laminaria hyperborean.*

8. The substance for use according to anyone of claims 1-7, wherein the one or more lactic acid bacterial strain is selected from the group consisting of the genus Enterococcus, Lactobacillus, Pediococcus or Lactococcus, or combinations thereof.

9. A substance for use according to anyone of claims 1-8, wherein the essentially homofermentative process is an essentially homolactic fermentation process.

10. A substance for use according to anyone of claims 1-9, wherein the fermented composition is provided by an anaerobic fermentation process.

11. A substance for use according to anyone of claims 1-10, wherein the content of plant material is at least 50%.

12. A substance for use according to anyone of claims 1-11, wherein the fermented composition is a prebiotic and a probiotic composition.


**Patentansprüche**

1. Substanz zur Verwendung bei der Behandlung, Linderung, Stabilisierung oder Prophylaxe einer autoinflammatorischen Erkrankung bei einem Säuger (wie einem Menschen oder einem Tier), wobei die Substanz eine fermentierte Zusammensetzung umfassend eine Kombination aus Brassica spp. und Seegras oder Algen; und einen oder mehrere Milchsäurebakterienstämme umfasst, wobei das Seegras oder die Algen Braunalgen sind und wobei die fermentierte Zusammensetzung durch einen im Wesentlichen homofermentativen Prozess bereitgestellt wird.

2. Substanz zur Verwendung nach Anspruch 1, wobei die autoinflammatorische Erkrankung bei einem Säuger ausgewählt ist aus der Gruppe bestehend aus Spondyloarthropathie, Spondylarthritis (insbesondere Psoriasisarthropathie oder Psoreasisarthropathie); Sklerose (wie systemische Sklerose); Allergie oder Intoleranz (wie Glutenallergie/-intoleranz und/oder Laktoseallergie/-intoleranz); Verstopfung; Müdigkeit (wie chronisches Müdigkeitssyndrom), Entzündung, Diarrhö; Darmerkrankungen (wie Morbus Crohn oder Cholitis ulcerosa bei einem Säuger), Hauterkrankungen (wie Pustulosis palmaris et plantaris, Akne, Rosacea und/oder Rhinophym) und/oder Rückenschmerzen mit und ohne röntgenologisch nachweisbare Modic-Veränderungen (I und II).

3. Substanz zur Verwendung nach einem der Ansprüche 1 oder 2, wobei die autoinflammatorische Erkrankung bei einem Säuger ausgewählt ist aus der Gruppe bestehend aus Psoriasis, Plaque-Psoriasis, Guttat-Psoriasis, inverser Psoriasis, pustulöser Psoriasis und erythrodermischer Psoriasis, Sklerose, wie systemischer Sklerose; Allergie oder Intoleranz (wie Glutenallergie/-intoleranz und/oder Laktoseallergie/-intoleranz) oder entzündlichen Darmerkrankungen (wie Morbus Crohn oder Cholitis ulcerosa).

4. Substanz zur Verwendung nach einem der Ansprüche 1-3, wobei die Substanz ein Adjuvans-Produkt ist, das von einem oder mehreren anderen Medikamenten begleitet wird.

5. Substanz zur Verwendung nach einem der Ansprüche 1-4, wobei die fermentierte Zusammensetzung einen pH-Wert unterhalb von 6,5, wie unterhalb von 6,0, z. B. unterhalb von 5,5, wie unterhalb von 5,0, z. B. im Bereich von 3,0-6,5, wie im Bereich von 3,0-6,0, z. B. im Bereich von 3,1-5,5, wie im Bereich von 5,0-3,2, wie im Bereich von 3,3-4-2, wie 3,4-4,0, wie 3,5-3,8, wie 3,7-4,2, wie 3,7-4,0 oder wie 3,8-4,2 aufweist.

6. Substanz zur Verwendung nach einem der Ansprüche 1-5, wobei die fermentierte Zusammensetzung eine Milchsäurekonzentration von mindestens 50 mM, wie mindestens 100 mM, wie 100-1000 mM, wie 100-500 mM, wie 100-300 mM, wie 100-200 mM, wie 150-500 mM, wie 200-500 mM oder wie 300-500 mM Milchsäure aufweist.

7. Substanz zur Verwendung nach einem der Ansprüche 1-6, wobei die Braunalgen ausgewählt sind aus Seetang, *Laminaria saccharina, Laminaria digitata* und/oder *Laminaria hyperborea.*

8. Substanz zur Verwendung nach einem der Ansprüche 1-7, wobei der eine oder die mehreren Milchsäurebakterienstämme ausgewählt sind aus der Gruppe bestehend aus der Gattung Enterococcus, Lactobacillus, Pediococcus oder Lactococcus oder Kombinationen davon.

9. Substanz zur Verwendung nach einem der Ansprüche 1-8, wobei der im Wesentlichen homofermentative Prozess ein im Wesentlichen homolaktischer Fermentationsprozess ist.

10. Substanz zur Verwendung nach einem der Ansprüche 1-9, wobei die fermentierte Zusammensetzung durch ein anaerobes Fermentationsverfahren bereitgestellt wird.

11. Substanz zur Verwendung nach einem der Ansprüche 1-10, wobei der Gehalt an Pflanzenmaterial mindestens 50 % beträgt.

12. Substanz zur Verwendung nach einem der Ansprüche 1-11, wobei die fermentierte Zusammensetzung eine präbiotische und eine probiotische Zusammensetzung ist.

**Revendications**

1. Substance pour une utilisation dans le traitement, le soulagement, la stabilisation ou la prophylaxie d'une maladie auto-inflammatoire chez un mammifère (tel qu'un humain ou un animal), ladite substance comprenant une composition fermentée comprenant une combinaison de Brassica spp. et d'algues marines ou d'algues ; et une ou plusieurs souches bactériennes d'acide lactique, dans laquelle l'algue marine ou l'algue est une algue brune et la composition fermentée est fournie par un procédé essentiellement homofermentaire.

2. Substance pour une utilisation selon la revendication 1, dans laquelle la maladie auto-inflammatoire chez un mammifère est choisie dans le groupe constitué de la spondylarthropathie, de la spondylarthrite (en particulier la psoriasisartropétie ou la psorasisartropie) ; de la sclérose (telle que la sclérodermie systémique) ; de l'allergie ou de l'intolérance (telle que l'allergie/intolérance au gluten et/ou l'allergie/intolérance au lactose) ; de la constipation ; de la fatigue (telle que le syndrome de fatigue chronique), de l'inflammation, de la diarrhée ; des maladies inflammatoires chroniques de l'intestin (telles que la maladie de Crohn ou la cholite ulcéreuse chez un mammifère), des maladies de la peau (telles que la pustulose palmo-plantaire, l'acné, la rosacée et/ou le rinophyma) et/ou des douleurs dorsales avec et sans modifications Modic vérifiées par radiographie (I et II).

3. Substance pour une utilisation selon l'une quelconque des revendications 1 ou 2, dans laquelle la maladie auto-inflammatoire chez un mammifère est choisie dans le groupe constitué du psoriasis, du psoriasis en plaques, du psoriasis en gouttes, du psoriasis inverse, du psoriasis pustuleux et du psoriasis érythrodermique, de la sclérose, telle que la sclérose systémique ; de l'allergie ou de l'intolérance (telle que l'allergie/l'intolérance au gluten et/ou l'allergie/l'intolérance au lactose) ou des maladies inflammatoires chroniques de l'intestin (telles que la maladie de Crohn ou la cholite ulcéreuse).

4. Substance pour une utilisation selon l'une quelconque des revendications 1 à 3, dans laquelle la substance est un produit adjuvant accompagné d'un ou de plusieurs autres médicaments.

5. Substance pour une utilisation selon l'une quelconque des revendications 1 à 4, dans laquelle la composition fermentée a un pH inférieur à 6,5, tel qu'inférieur à 6,0, par exemple inférieur à 5,5, tel qu'inférieur à 5,0, par exemple dans la plage de pH 3,0 à 6,5, tel que dans la plage de pH 3,0 à 6,0, par exemple dans la plage de pH 3,1 à 5,5, tel que dans la plage de pH 5,0 à 3,2, tel que dans la plage de 3,3 à 4,2, tel que 3,4 à 4,0, tel que 3,5 à 3,8, tel que 3,7 à 4,2, tel que 3,7 à 4,0, ou tel que 3,8 à 4,2.

6. Substance pour une utilisation selon l'une quelconque des revendications 1 à 5, dans laquelle la composition fermentée a une concentration en acide lactique d'au moins 50 mM, telle qu'au moins 100 mM, telle que 100 à 1 000 mM, telle que 100 à 500 mM, telle que 100 à 300 mM, telle que 100 à 200 mM, telle que 150 à 500 mM, telle que 200 à 500 mM ou telle que 300 à 500 mM d'acide lactique.

7. Substance pour une utilisation selon l'une quelconque des revendications 1 à 6, dans laquelle l'algue brune est choisie parmi les algues, *Laminaria saccharina, Laminaria digitata* et/ou *Laminaria hyperborean.*

8. Substance pour une utilisation selon l'une quelconque des revendications 1 à 7, dans laquelle l'au moins une souche bactérienne d'acide lactique est choisie dans le groupe constitué du genre Enterococcus, Lactobacillus, Pediococcus ou Lactococcus, ou des combinaisons de ceux-ci.

9. Substance pour une utilisation selon l'une quelconque des revendications 1 à 8, dans laquelle le processus essentiellement homofermentaire est un processus de fermentation essentiellement homolactique.

10. Substance pour une utilisation selon l'une quelconque des revendications 1 à 9, dans laquelle la composition fermentée est fournie par un procédé de fermentation anaérobie.

11. Substance pour une utilisation selon l'une quelconque des revendications 1 à 10, dans laquelle la teneur en matière végétale est d'au moins 50 %.

12. Substance pour une utilisation selon l'une quelconque des revendications 1 à 11, dans laquelle la composition fermentée est une composition pré-biotique et pro-biotique.

Fig. 1

EP 3 370 750 B1

Fig. 2

EP 3 370 750 B1

Fig. 3

29

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014131422 A
- WO 2014206419 A
- WO 2013029632 A

**Non-patent literature cited in the description**

- **JIN-WOO JEONG et al.** *Journal of Medicinal Food*, 2015, vol. 18 (6), 677-684
- **HYUN JU KIM et al.** *J. Agric. Food Chem.*, 2007, vol. 55, 10486-10492
- **JEONG SOOK NOH et al.** *British Journal of Nutrition*, 2013, vol. 109, 17-24
- **YE-RANG YUN et al.** *Journal of Medicinal Food*, 2014, vol. 17 (8), 886-893